# EUROPÄISCHE PATENTSCHRIFT

(11) **EP 1 951 314 B1**
(45) Veröffentlichungstag und Bekanntmachung des Hinweises auf die Patenterteilung: **28.09.2011**
(21) Anmeldenummer: 06828863.8
(22) Anmeldetag: 25.10.2006
(51) Int. Cl.: A61K 47/48, A61K 9/16

(54) **DRUG DELIVERY SYSTEME**
DRUG-DELIVERY SYSTEMS
SYSTEME D'ADMINISTRATION DE MEDICAMENTS

(30) Priorität: 25.10.2005 DE 102005051366
(43) Veröffentlichungstag der Anmeldung: 06.08.2008
(73) Patentinhaber: Evonik Röhm GmbH, 64293 Darmstadt (DE)
(72) Erfinder: SEILER, Matthias, 64347 Griesheim (DE); WINDHAB, Norbert, 65719 Hofheim (DE); STICKLER, Manfred, 64342 Seeheim-Jugenheim (DE); PETEREIT, Hans-Ulrich, 64291 Darmstadt (DE)
(86) Internationale Anmeldenummer: PCT/EP2006/010301
(87) Internationale Veröffentlichungsnummer: WO 2007/048599

(56) Entgegenhaltungen:
- EP-A- 1 352 657
- WO-A-02/11772
- WO-A-03/051281
- WO-A-2004/072153
- WO-A-2005/055949
- WO-A-2007/055561
- KOHLE: "Drug complexation..." INT. J. OF PHARMACEUTICALS 259 (2003) 143-160, 2003, XP008086791
- DE LONG: "Characterization of complexes of oligonucleaotides..." J. OF PHARMACEUTICAL SCIENCES 86, 6, 762-764, 1997, XP008086807

## Beschreibung

Die Erfindung betrifft neuartige Drug Delivery Systeme gemäß den Ansprüchen, basierend auf polymeren Trägern, insbesondere verzweigten, vernetzten oder dendritischen Trägem die sowohl mindestens einen Wirkstoff als auch mindestens eine Signalsubstanz enthalten, wobei Träger, Wirkstoff und Signalsubstanz nicht spezifisch miteinander verknüpft oder bindend komplementär sind.

Eines der größten Probleme bei der medikamentösen Behandlung von Krankheiten stellt der gezielte Transport von Wirkstoffen in den erkrankten Zielort, also in ein Gewebe, ein Organ bzw. in die entsprechenden Zellen dar. Membranen sind dabei die wichtigsten Barrieren, die den Zielort (Wirkort) gegenüber den zu transportierenden Wirksubstanzen abschirmen. Ein weiteres Problem ist der Abbau oder die derivatisierung freier Wirkstoffe im Organismus. Eine solche Metabolisierung vermindert oder vereitelt oft eine gezielte pharmakologische Wirkung der Wirkstoffe am Zielort. Darüber hinaus können im Körperfalsch verteilte bzw. veränderte Wirkstoffe, insbesondere wenn sie lokal oder systemisch toxisch sind, zu unerwünschten Nebenwirkungen führen.

Ein bereits erprobter Weg diese Nachteile zu umgehen besteht in der Herstellung von partikulären Wirkstoffformulierungen, wobei der Wirkstoff in eine polymere Hülle oder Matrix eingebunden vorliegt (Nishiyama et al., Drug Discovery Today: Technologies (2005), 2(1), 21-26. Publisher: Elsevier B.V.). Allgemein können dabei reine Volumen-Carrier, bei denen der Wirkstoff in einer Art polymeren Container-Vesikel eingeschlossen ist, von chemisch-funktionalisierten polymeren Carrieren unterschieden werden, bei denen einzelne Inhaltsstoffe, z.B. Wirkstoffe oder Signalsubstanzen chemisch an den funktionalisierten Träger gebunden sind (Matrix-Typ). EP 1352657 offenbart zusammensetzungen, welche Lactoferrin, Stärke und Laktose enthalten.

Der Transport der Wirkstoffe in das erkrankte Gewebe erfolgt im Falle bloßer Träger/Wirkstoff-Formulierungen durch einfache Freisetzung und Diffusion (Equilibrierung). Um die Zielsteuerung bestimmter Wirkstoffe zu verbessern werden häufig Signalstoffe kovalent bzw. koordinativ an den Wirkstoff bzw. an den Träger gebunden, wobei die Signalsubstanz spezifisch an Zellmembranen des erkrankten Gewebes bindet und die endocytotisch beschriebene Aufnahme des Wirkstoffes in die Zelle initiiert (z.B. WO 2005/084158, WO 20041072153, Pitard, B. et al., Proceedings of the National Academy of Sciences of the United States of America (1999), 96(6), 2621-2626).

Durch die Anbindung von Signalstoffen, die die endocytotische Aufnahme des Wirkstoffes oder des partikulären Transportsystems einleiten, kann zwar die Zielsteuerung des Wirkstoffes gegenüber der rein diffusionsartigen Aufnahme verbessert werden, allerdings werden durch die chemische Funktionalisierung des Trägers bzw. durch die kovalente oder koordinative Anbindung des Signalstoffes an den Wirkstoff auch neue Probleme erzeugt.

So müssen insbesondere Arzneimittelwirkstoffe im Hinblick auf ihre Absorption, Verteilung, Metabolisierung und Ausscheidung (ADME-Parameter) sowie im Hinblick auf ihre Wirkung und Toxizität optimiert werden. Durch die Anbindung eines Signalstoffes an den Wirkstoff werden diese Eigenschaften häufig verändert, der Wirkstoff kann dadurch in seiner pharmazeutischen Verwendbarkeit eingeschränkt oder sogar unbrauchbar werden. Die Bindung eines Wirkstoffes an einen funktionalisierten Träger wirft in aller Regel ähnliche Probleme auf. Zudem müssen Träger, Signalstoff bzw. Wirkstoff häufig extra funktionalisiert werden, um eine entsprechende spezifische Anbindung zu ermöglichen. Solche chemisch funktionalisierten Träger und Signalstoffe lassen folglich nur eingeschränkte Träger, Signalstoff, Wirkstoff -Kombinationen zu. Darüber hinaus sind diese Drug-Delivery-Systeme aufwendig herzustellen und der Wirkstoff muss in der Regel, sofern er an den Signalstoff oder den Träger kovalent oder koordinativ gebunden ist, am Zielort chemisch freigesetzt werden. Weiterhin entsteht durch eine kovalente oder koordinative Anbindung eines Signalstoffes oder eines Trägers ein neuer chemischer Wirkstoff, der eine aufwendige klinische Erprobung erfordert.

Ziel der vorliegenden Erfindung war es nun neuartige Drug-Delivery-Systeme bereitzustellen, die den zielgerichteten Transport des Wirkstoffes durch biologische Barrieren ermöglichen und die zumindest einen Teil der genannten Probleme beheben.

Dabei wurde überraschend festgestellt, dass es ausreicht partikuläre Drug-Delivery-Systeme basierend auf einem verzweigten bzw. vernetzten polymeren Träger zu verwenden, die sowohl mit den jeweilig eingesetzten Wirkstoffen als auch mit den verwendeten Signalsubstanzen unspezifisch aggregiert sind, ohne dass eine kovalente oder koordinative Anbindung der Signalsubstanz an den Wirkstoff oder den Träger notwendig ist.

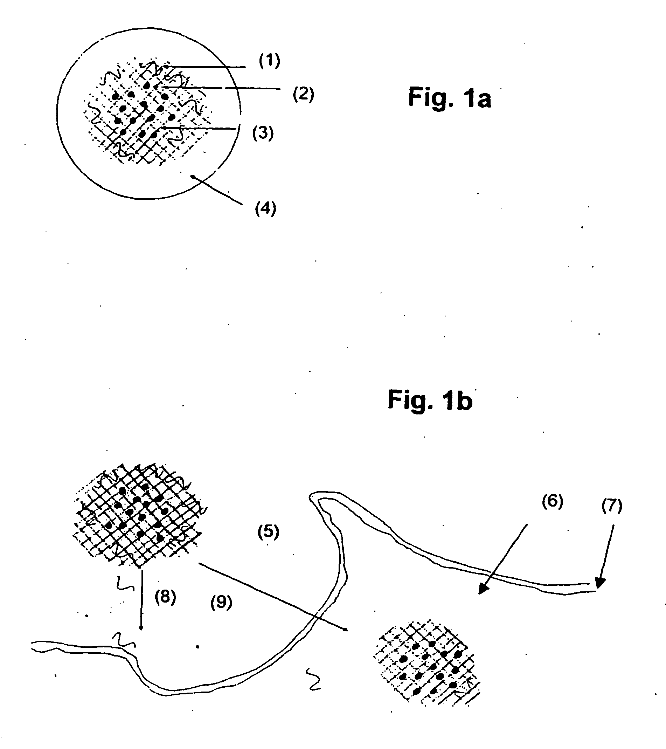

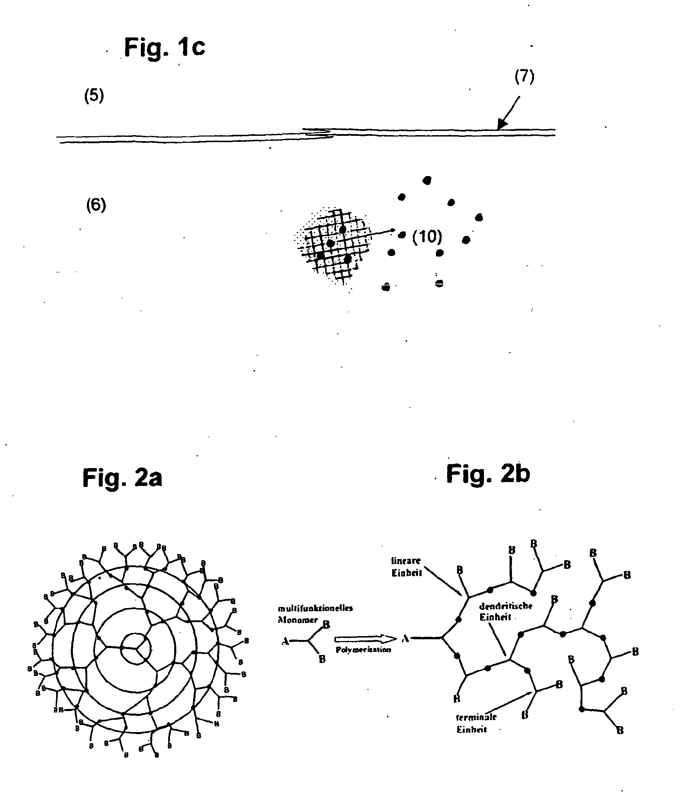

Auch wenn die bisher diskutierten endozytotischen und diffusiven Modelle den Wirkstofftransport durch biologische Membranen nicht widerspruchsfrei erklären konnten, wurde bisher stillschweigend davon ausgegangen, dass für einen endocytotischen Transport eines Wirkstoffes durch eine Zellmembran eine spezifische Verknüpfung zwischen dem Wirkstoff und dem Transport-initiierenden Signalstoff bzw. zwischen dem Signalstoff und weiteren Fusionseinheiten oder zwischen dem Signalstoff und dem Wirkstoff enthaltenden Träger bestehen muss, wobei im ersten Fall direkt der Wirkstoff und im zweiten Fall der Träger mit dem Wirkstoff in die Zelle transportiert wird. Gemäß der vorliegenden Erfindung ist aber gerade eine solche spezifische chemische Kopplung nicht nötig. Dieser Wirkstofftransport könnte dabei auf einen bisher nicht bekannten Mechanismus zur zellularen Aufnahme von Wirkstoffen zurückzuführen sein (Fig. 1).

Gegenstand der vorliegenden Erfindung sind Drug-Delivery-Systeme gemäß Anspruch 1.

Als Träger kommen lineare Polymere, wie z. B. Polylactide in Betracht. Bevorzugt sind allerdings polymere Träger, die mindestens ein verzweigtes oder vernetztes Polymer enthalten, da verzweigte oder vemetzte Polymere für eine bloße Aggregierung von Signalstoff, Wirkstoff und Träger besonders geeignet sind. Der Anteil der verzweigten bzw. vemetzten Trägerpolymere beträgt bevorzugt über 10 Gew.-%, insbesonder über 50 Gew.-% bezogen auf das Gesamtgewicht des Trägers.

Die Signal- und Wirkstoffe liegen dabei bevorzugt im polymeren Träger dispergiert bzw. koazerviert vor. Insbesondere eignen sich dazu dendritische oder hochvemetzte Polymere sowie Kammpolymere. Dabei sind naturidentische oder naturisomere Polymere von besonderem Interesse.

Besonders bevorzugte Träger sind die in der Fachliteratur auch als "dendritische Polymere" bezeichneten hochverzweigten, globularen Polymere. Diese aus multifunktionellen Monomeren synthetisierten dendritischen Polymere lassen sich in zwei unterschiedliche Kategorien einteilen, die "Dendrimere" sowie die "hyperverzweigten Polymere". Dendrimere besitzen einen sehr regelmäßigen, radialsymmetrischen Generationenaufbau. Sie stellen monodisperse globulare Polymere dar, die im Vergleich zu hyperverzweigten Polymeren in Vielschritt-Synthesen hergestellt werden. Dabei ist die Struktur durch drei unterschiedliche Areale charakterisiert:
- den polyfunktionellen Kern, der das Symmetriezentrum darstellt,
- verschiedenen wohldefinierten radialsymmetrischen Schichten einer Wiederholungseinheit (Generation) und
- den terminalen Guppen.

Die hyperverzweigten Polymere sind im Gegensatz zu den Dendrimeren polydispers und hinsichtlich ihrer Verzweigung und Struktur unregelmäßig. Neben den dendritischen und linearen Einheiten treten - im Gegensatz zu Dendrimeren - in hyperverzweigten Polymeren auch lineare Einheiten auf. Jeweils ein Beispiel für ein Dendrimer (Fig. 2a) und ein hyperverzweigtes Polymer (Fig. 2b), aufgebaut aus Wiederholungseinheiten, welche jeweils drei Bindungsmöglichkeiten aufweisen, ist in Fig. 2 schematisch dargestellt. Wobei die hier verwendeten dendritischen Polymere mindestens 3 Wiederholungseinheiten pro Molekül, bevorzugt mindestens 10 Wiederholungseinheiten pro Molekül und besonders bevorzugt mindestens 100 Wiederholungseinheiten pro Molekül und ganz besonders bevorzugt mindestens 200 Wiederholungseinheiten pro Molekül oder noch besser mindestens 400 Wiederholungseinheiten pro Molekül aufweisen, die wiederum jeweils mindestens drei, bevorzugt mindestens vier Bindungsmöglichkeiten aufweisen, wobei mindestens 3 dieser Wiederholungseinheiten, besonders bevorzugt mindestens 10 und darüber hinaus bevorzugt mindestens 20 jeweils über mindestens drei, bevorzugt über mindestens vier Bindungsmöglichkeiten mit mindestens drei, bevorzugt mindestens vier weiteren Wiederholungseinheiten verknüpft sind. Üblicherweise weisen die hyperverzweigten Polymere maximal 10000, vorzugsweise maximal 5000 und besonders bevorzugt maximal 2500 Weiderholungseinheiten auf.

In einer bevorzugten Ausführungsform weist das hochverzweigte dendritische Polymer mindestens drei Wiederholungseinheiten auf, welche jeweils mindestens drei mögliche Bindungsmöglichkeiten aufweisen, wobei mindestens drei dieser Wiederholungseinheiten mindestens zwei mögliche Bindungsmöglichkeiten aufweisen.

Dabei wird unter dem Begriff "Wiederholungseinheit" vorzugsweise eine stets wiederkehrende Struktur innerhalb des hyperverzweigten Moleküls, z.B. lineare, dendritische oder terminale Einheiten wie sie in Seiler, Fortschritt-Berichte VDI, Reihe 3, Nr. 820 ISBN 3-18-382003-x und Gao, C. et al., Hyperbranched Polymers: from synthesis to application, Prog. Polym. Sci., 29 (2004) 183-275 definiert ist, verstanden. Unter dem Begriff "Bindungsmöglichkeit" wird vorzugsweise diejenige funktionelle Struktur innerhalb einer Wiederholungseinheit verstanden, mit der eine Verknüpfung zu einer anderen Wiederholungseinheit möglich ist. Bezogen auf die vorstehend dargestellten Beispiele eines Dendrimers bzw. hyperverzweigten Polymers ist die Wiederholungseinheit eine Struktur mit jeweils drei Bindungsmöglichkeiten (X,Y,Z):

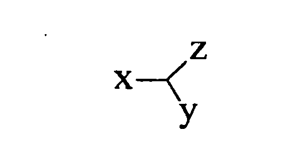

Die Verknüpfung der einzelnen Bindungseinheiten miteinander kann durch Kondensationspolymerisation, durch radikalische Polymerisation, durch anionische Polymerisation, durch kationische Polymerisation, durch Gruppentransferpolymerisation, durch koordinative Polymerisation, durch Ringöffnungspolymerisation oder durch enzymatisch katalysierte Polymerisation erfolgen.

Besonders bevorzugte Dendrimere sind Starbust® Polyamidoamin (PAMAM)-Dendrimere, Polypropylenimin-Dendrimere, Polyethylenoxid-basierte Dendrimere, Polyether-Dendrimere, beschichtete PAMAM-Dendrimere, z.B. mit Polylactid-coglycolid-Beschichtung, Polylysin-Dendrimere, darunter auch Polylysin-block-PEGblock-Polylysin-Dendrimere, und Polyarylether. Solche bevorzugten Dendrimere sind z.B. beschrieben in Frechet, J.M.J. et al., Dendrimers and Other Dendritic Polymers, John Wiley & Sons Ltd., West Sussex, UK (2001); Malik, N. et al., Journal of Controlled Release 65, (2000), 133-148; Frey, H. et al., Reviews in Molecular Biotechnology 90 (2002) 257-267; Jikei, M. et al., Hyperbranched Polymers: a promising new class of materials, Prog. Polym. Sci., 26 (2001), 1233-1285.

In diesem Zusammenhang bevorzugte lineare oder hyperverzweigte Trägerpolymere sind Polyester, Polyesteramide, Polyether, Polyamide, Polyethylenimine, Polyglycerine, Polyglycolide, Polylactide, Polylactid-co-glycolide, Polytartrate und Polysaccharide. Unter diesen Polymeren besonders bevorzugt sind die bereits kommerziell unter der Marke Boltom^{®} bei der Firma Perstorp AB erhältlichen hyperverzweigten Polyester, die unter der Marke Hybrane^{®} bei der Firma DSM BV Niederlande erhältlichen hyperverzweigten Polyesteramide, die von der Firma Hyperpolymers GmbH hergestellten Polyglycerine sowie die als Polyimin^{®} bei der Firma BASF AG erhältlichen hyperverzweigten Polyethylenimine.

Weiterhin bevorzugte verzweigte Trägerpolymere sind Polycaprolactone, Copolymere wie Poly(D,L-lactid-co-glycolide) sowie die von der Degussa AG hergestellten Polyesterverbindungen aus den Produktfamilien Dynapol^{®}S und Dynacoll^{®}.

Besonders bevorzugte dendritische Polymere sind Polymere mit einer Molmasse zwischen 1000 g/mol und 2 000 000 g/mol, besonders bevorzugt zwischen 2000 g/mol und 700 000 g/mol und ganz besonders bevorzugt zwischen 6000 g/mol und 100 000 g/mol, einer Schmelztemperatur bevorzugt zwischen 0 °C und 150 °C und/oder einer Schmelzviskosität der bevorzugten Trägerpolymere von kleiner als 3,0 Pas, bevorzugt kleiner 2,5 Pas, insbesondere kleiner 2,0 Pas, gemessen bei 80 °C. Insbesondere hyperverzweigte polymere Träger besitzen darüber hinaus einen Verzweigungsgrad zwischen 20% und 100 %, bevorzugt zwischen 30% und 70%, und/oder eine Hydroxy-Zahl zwischen 10 mg KOH/g und 600 mg KOH/g. Der Verzweigungsgrad der Dendrimere liegt bevorzugt zwischen 25% und 75%.

Bevorzugt sind als Trägerpolymere weiterhin verzweigte oder vemetzte Homo- oder Heteropolymere aus Kohlenhydraten (Polysacchariden); aus natürlichen und artifiziellen Aminosäuren; aus natürlichen und artifiziellen Nukleinsäuren; aus Polyaminen; aus Polyestern; aus Polyethern; aus Polyolen, insbesondere Polyvinylalkoholen, aus Polyolefinen, insbesondere aus Polyisoprenen, Polyethylenen, Polypropylenen, Polybutadienen oder Polystyrolen; aus Polyalkylenglykolen, insbesondere aus Polyethylenglykolen, aus Polyamiden, aus Polyacetalen, aus Polyacrylaten; aus Polyacetaten, insbesondere aus Polyvinylacetaten; aus Polyurethanen; aus siliziumorganischen Polymeren, wie z.B. Siliconen; aus Epoxidharzen, aus Polythiolen oder aus Polycarbonaten. Bevorzugte polymere Träger sind weiterhin biokompatibel und verzögert enzymatisch abbaubar. Hierzu sind insbesondere enzymatisch abbaubare verzweigte oder vemetzte Trägerpolymere aus der Gruppe der Polycaprolactone, Polyglycolide, Polylactide, Polylactid-co-glycolide, Polytartrate oder Polyester zu nennen. Besonders bevorzugt sind verzweigte oder vemetzte Polysaccharide, auf Basis von Cellulose, Pektin, Amylopektin oder Dextranen.

Besonders bevorzugte vemetzte Trägerpolymere sind Hydrogele, insbesondere dendritische Hydrogele, wie sie z.B. in Rueda, J. C., et al. Macromol. Chem. Phys. 2003, 204, 947-953; Hatice, K.C. et al., published online 24. October 2003 in Wiley InterScience DOI 10.1002/app.13125; Knischka, R. et al., Polymeric Materials: Science & Engineering 2001, 84, 945, beschrieben sind.

In einer besonders bevorzugten Ausführungsform werden polymere Träger verwendet, die möglichst naturbelassen oder naturnah sind und insbesondere nicht weiter funktionalisiert bzw. derivatisiert sind. Zum einen kann so sichergestellt werden, dass keine ungewünschten chemischen Reaktionen mit den Wirkstoffen bzw. den Signalsubstanzen ablaufen, wodurch deren pharmokologisches Eigenschaftsprofil verändert werden könnte, zum anderen wird so die Gefahr einer allergischen Reaktion auf das Drug-Delivery-System minimiert.

Die verzweigten oder vemetzten Polymere besitzen in einer bevorzugten Ausführungsform einen Anteil von mindestens 50 Gew.-%, bevorzugt von über 75 Gew.-% bezogen auf das Gesamtgewicht des Trägers. Darüber hinaus können weitere Polymere, insbesondere auch unverzweigte und nicht vernetzte Polymere der eben genannten Polymerklassen dem Träger beigemischt werden. In einer besonders bevorzugten Ausführungsform werden ausschließlich verzweigte oder vernetze Polymere als Träger eingesetzt.

Der Anteil des Trägers an dem beanspruchten Drug-Delivery-System beträgt bevorzugt zwischen 30 Gew.-% und 99,5 Gew.-%; bevorzugt zwischen 50 Gew-% und 98 Gew-% bezogen auf das Gesamtgewicht des partikulären Drug-Delivery-Systems. Wobei Formulierungen mit hochaktiven Wirkstoffen bevorzugt einen Trägeranteil von über 80 Gew.-% bis zu 99,5 Gew.-%, insbesondere zwischen 90 Gew.-% und 99 Gew.-%, aufweisen. Formulierungen mit üblichen Wirkstoffen besitzen demgegenüber einen bevorzugten Trägeranteil von 55 Gew.-% bis 94,5 Gew. %, besonders bevorzugt zwischen 65 Gew.-% und 94 Gew.-%.

Als Signalstoffe oder Signalsubstanzen im Sinne der vorliegenden Erfindung sind alle Stoffe zu verstehen, die einen zielgerichteten Transport eines Wirkstoffes durch eine biologische Barriere initiiren können und die unter die Ansprüche: fallen.

Erfindungsgemäße Signalpeptide sind Peptide mit einer Aminosäurensequenz
KCFQWQRNMRKVRGPPVSCIKR (SEQ ID No. 3) oder
CFQWQRNMRKVRGPPVSC (SEQ ID No. 4),

Eine weitere bevorzugte Ausführungsform sind Derivate von Peptiden mit einer Aminosäuresequenz gemäß SEQ ID No. 3 oder 4, wobei der Methioninrest durch eine Aminosäure ausgewählt aus der Gruppe umfassend Valin, Isoleucin, Norvalin, Leucin und Norleucin ausgetauscht ist. Solche Peptide sind z.B. Peptide mit einer Aminosäurensequenz:
KCFQWQRNVRKVRGPPVSCIKR (SEQ ID No. 7),
KCFQWQRNIRKVRGPPVSCIKR (SEQ ID No. 8),
KCFQWQRNXRKVRGPPVSCIKR (SEQ ID No. 9), wobei X Norvalin ist,
KCFQWQRNLRKVRGPPVSCIKR (SEQ ID No. 10),
KCFQWQRNXRKVRGPPVSCIKR (SEQ ID No. 28), wobei X Norleucin ist,
CFQWQRNVRKVRGPPVSC (SEQ ID No. 11),
CFQWQRNIRKVRGPPVSC (SEQ ID No. 12),
CFQWQRNXRKVRGPPVSC (SEQ ID No. 13), wobei X Norvalin ist,
CFQWQRNLRKVRGPPVSC (SEQ ID No. 14);
CFQWQRNXRKVRGPPVSC (SEQ ID No. 15), wobei X Norleucin ist.

Die genannten peptidischen Signalstoffe können radioaktiv markiert sein, bevorzugt mit einer radioaktiv-markierten Aminosäure, insbesondere mit einer Tritium-markierten Aminosäure. Darüber hinaus können die Signalstoffe auch mit detektierbaren Gruppen modifiziert sein, wobei solche Gruppen bevorzugt ausgewählt sind aus der Gruppe der Fluorophore, der radioaktiven Tracer und Haptene, wobei das Hapten Biotin besonders geeignet ist.

Durch den Einsatz humaner Signalstoffe, insbesondere der erfindungsgemäßen von Lactoferrin abgeleiteten zellpenetrierenden Peptide, können insbesondere allergische Reaktionen gerichtet gegen das Drug-Delivery-System minimiert werden. Weiterhin zeigen die von Lactoferrin abgeleiteten Peptide eine hohe Effizienz bei der Zellpenetration sowohl im Hinblick auf die in die Zelle aufgenommene Menge an Peptid bzw. Peptidformulierung als auch im Hinblick auf die zur Aufnahme benötigten Zeit Weiterhin vorteilhaft ist, dass die aufgenommenen Peptide, Formulierungen bzw. Bestandteile der Formulierungen in das Cytoplasma der Zelle transportiert werden.

Die erfindungsgemäßen vom Lactoferrin abgeleiteten Signalpeptide sind insbesondere im Hinblick auf den Wirkstofftransport durch das Darmepithel von Interesse, wobei der Wirkstoff mit hoher Effizienz in die Epithelzellen transportiert wird und im Anschluss in die Blutbahn abgegeben wird. Für einen zielgerichteten Transport von Wirkstoffen, z.B. in Tumorzellen, kann das Signalpeptid auch maskiert sein. Über eine proteolitische Spaltung, z.B. durch Proteasen, die von Tumorzellen freigesetzt werden, kann dann das maskierte Peptid in seine funktionelle zellpenetrierende Form gespalten werden (analog Jiang, T. et al., Proc. Natl. Acad. Sci., USA, 17867-17872, 2004).

Weiterhin können die genannten Signalpeptide mit üblichen Verfahren derivatisiert vorliegen. Insbesondere sind hierbei C-terminal acetylierte oder amidierte Peptide zu erwähnen, die sich oft durch eine gute Stabilität unter physiologischen Bedingungen auszeichnen. Bevorzugt werden allerdings unmodifizierte Signalpeptide verwendet.

Die Beladung des Trägers mit einem Signalstoff erfolgt typischerweise mit einer Menge zwischen 0,5 Gew.-% und 20 Gew.-%, bevorzugt zwischen 1 Gew.% und 10 Gew.-%, bezogen auf das Gesamtgewicht des partikulären Drug-Delivery-Systems.

Weiterhin ist besonders aus der "Antisense-Technologie bekannt, dass ionische Verbindungen, Nukleinsäuren, Nukleinsäurefragmente, Nukleinsäurekonjugate und Nukleinsäureanaloga Membrantransporte verstärken können, d.h. reine oder chemisch modifizierte Fragmente ebenfalls als ergänzender Stimulus eingesetzt werden.

Da keine spezifische Ankopplung, weder der Wirkstoffe noch der Signalstoffe, erfolgt, ist die Beladung des polymeren Trägers im Hinblick auf die verwendbaren Wirk- und Signalstoffe zur Herstellung der partikulären Drug-Delivery-Systeme nicht beschränkt. Als besonders vorteilhaft sind die hier beschriebenen Transportsysteme allerdings für Wirkstoffe oder Wirkstoffmischungen anzusehen, die vor einem chemischen oder enzymatischen Abbau während des Transports zum Zielort geschützt werden müssen bzw. die zur Vermeidung von Nebenwirkungen erst am Zielort freigesetzt werden dürfen. Folglich sind die beschriebenen Drug-Delivery-Systeme insbesondere zum Transport pharmazeutisch wirksamer Peptide oder Proteine geeignet. Solche Wirkstoffe sind z.B. Pharmaproteine oder Proteowirkstoffe, wie z.B. Antikörper, peptidischer Hormone, Rezeptoren bzw. deren peptidische Liganden oder Enzyme. Beispielhaft seien hierfür angeführt der α1-Protease-Inhibitor, Eglin, Elastase, α1-Antitrypsin (Emphyseme), Antithrombin (Anticoagulans), Angiotensinase (Bluthochdruck), Faktor VII, VIII, IX, X, Fibrinogen, Thrombin, Plasminogen Activator Inhibitor (Blutgerinnungsstörungen), Immunglobuline (Passive Immunisierung), Gancyclovir, Acyclovir, Interferone (Virusinfektionen, Tumortherapie), Tumor-Nekrose-Fäktor, Cachectin, Dihydrofolat-Reduktase, Lymphotoxin, Interleukine, Tumor-Suppressor Proteine, wie z.B. p53 (Krebs), Plasmin, Urokinase, Hirudin, Streptokinase, Urokinase, Tissue Plasminogen Activator, Protein C, Protein S (Thrombolyse), Phospholipase A₂, Uromodulin, Tamm-Horsfall-Protein (Entzündungen), Insulin (Diabetis), Trypsin-Inhibitor (Pankreatitis), Lysozym, Thymopoietin, peptidische Antibiotika (bakterielle Infektionen), Erythropoietin (Anämie). Allerdings bleiben die hier beschriebenen Drug-Delivery-Systeme nicht auf solche Wirkstoffe beschränkt, es können auch niedermolekulare Wirkstoffe ("small molecules"), wie z.B. viele antivirale Substanzen, lebertherapeutische Substanzen, neuroprotektive Substanzen, Immuntherapeutika und -suppressiva, niedermolekulare Wirkstoffe gegen cardiovaskuläre Erkrankungen oder Krebs, Schmerzmittel, , niedermolekulare entzündungshemmende, antibiotische und antimikrobielle Wirkstoffe und niedermolekulare Hormone, oder makromolekularen Wirkstoffe, wie. z.B. Nukleinsäurefragmente oder Nukleinsäuren (genomische DNA, cDNA, mRNA, siRNA, antisense Oligonukleotide etc.), eingesetzt werden. Niedermolekulare Wirkstoffe sind z.B. Nucleosidanaloga, ß-Interferone, α-Liponsäure, Peptidanaloga, Enzym- bzw. Rezeptorinhibitoren, -agonisten und -antagonisten, Prostaglandine, Steroide, Cytostatika und heterozyklische Antibiotika. Insbesondere können auch Wirkstoffe in Form ihrer Prodrugs eingesetzt werden.

Ein Vorteil der vorliegenden Transportpartikel ist es, dass auch zwei oder mehr Wirkstoffe, insbesondere auch mit unterschiedlichen physikalischen Eigenschaften, wie z.B. unterschiedlicher Hydrophilie, zusammen in eine Formulierung eingearbeitet werden können. Eine pharmakologische Einteilung diesbezüglich bietet das von der FDA entwickelte BCS-System, das Arzneimittelwirkstoffe in vier unterschiedliche Klassen einteilt. Die hier beschriebenen Partikel sind in besonderem geeignet gerade Wirkstoffe aus unterschiedlichen Klassen in ein Transportsystem aufzunehmen.

Die Beladung des Trägers mit einem Wirkstoff erfolgt typischerweise mit einer Menge zwischen 0,001 Gew.-% bis 50 Gew.-% bezogen auf das Gesamtgewicht des partikulären Drug-Delivery-Systems. Wobei hochaktive Wirkstoffe bevorzugt in einer Menge zwischen 0,01 Gew.-% und 1 Gew.-%, besonders bevorzugt unter 0,1 Gew.-% enthalten sind. Andere Wirkstoffe werden im allgemeinen in einer Menge zwischen 1 Gew.-% und 30 Gew.-%, bevorzugt zwischen 5 Gew.-% und 25 Gew.-% eingesetzt werden.

Die Wirkstoffanteil im partikulären Drug-Delivery-System wird für pharmazeutische Formulierung so gewählt, dass bevorzugt zwischen 0,1 mg und 100 mg Wirkstoff pro kg Körpergewicht eines Patienten erreicht werden. Bei hochaktiven Wirkstoffen, wie z.B. Hormonen reichen auch niedrigere Dosierungen aus.

Ein besonders bevorzugtes Merkmal der hier verwendeten Signal- und Wirkstoffe ist, dass sie unmodifiziert sind. Unter "unmodifiziert" im Sinne der vorliegenden Erfindung wird dabei verstanden, dass der Signalstoff bzw. der Wirkstoff mit keinen zusätzlichen funktionellen Gruppen oder Linkem derivatisiert wird, um eine koordinative oder kovalente Bindung an den Träger oder untereinander zu ermöglichen.

Unter dem Begriff der "biologischen Barriere" im Sinne der vorliegenden Erfindung sind neben der Zellmembran im allgemeinen insbesondere auch Epithel- bzw. Endothelzelllagen zu verstehen. Folglich fallen auch Organ- bzw. Gewebeschranken, wie z.B. die Blut-Him-Schranke oder das Darmepithel unter den Begriff der biologischen Barrier. Wobei nach dem Durchschreiten der Barriere die Vaskularisierung für einen guten pharmakokinetischen Weitertransport (Wirkstoffsog) sorgt.

Entscheidend am Aufbau der erfindungsgemäßen partikulären Drug-Delivery-Systeme ist, dass sowohl Wirkstoffe als auch Signalstoffe unspezifisch aggregiert mit dem verzweigten oder vemetzten polymeren Trägern vorliegen, also keine kovalente Bindung untereinander eingehen. Unter den Begriff kovalente Bindung fallen auch koordinative Bindungen, wobei das bindende Elektronenpaar von einem Bindungspartner einem definierten Elektronenpaarakzeptor bereitgestellt wird. Durch den Begriff koordinative Bindung werden keine unspezifischen Bindungen umfasst, die unter physiologischen Bedingungen nicht stabil und nicht selektiv sind. Spezifische koordinative Bindungen stellen z.B. Komplexierungen, wie Epitop-Bindungen oder haptamerartige bzw. aptamerartige Bindungen dar. Auch allgemein als Wirts-Gast-Bindungen bezeichnete Verknüpfungen sind als eine komplexbildende Bindung anzusehen. Solche koordinativen Bindungen zeichnen sich durch ihre Stabilität und Selektivität der Bindung unter physiologischen Bindungen aus. Nicht selektive, unspezifische Bindungen im Sinne der vorliegenden Erfindung sind polare Wechselwirkungen bzw. lipophile Wechselwirkungen und Van der Waals Wechselwirkungen, die in der Regel eine Bindungsenthalpie von unter 50 kJ/mol bzw. von unter 20 kJ/mol besitzen (je betrachteten Rest der für eine solche Bindung zur Verfügung steht). In den hier beschriebenen partikulären Transportsystemen liegen demgegenüber sowohl die Signalstoffe als auch die entsprechenden Wirkstoffe bevorzugt einfach im polymeren Trägermaterial dispergiert bzw. koazerviert vor.

So gestaltete Drug-Delivery-Systeme besitzen vielfältige Vorteile-gegenüber herkömmlichen Wirkstoffformulierungen. So können die Wirkstoffe und die Signalsubstanzen ohne chemische Modifikationen eingesetzt werden, wodurch ihre Eigenschaften, insbesondere im Hinblick auf ihre Wirkung als auch bezüglich der ADME-Parameter erhalten. Weiterhin können auch natürliche oder aus natürlich vorkommenden Monomeren aufgebaute verzweigte oder vemetzte polymere Träger eingesetzt werden. So können gut verträgliche partikuläre Drug-Delivery-Systeme erhalten werden, die insbesondere auch zur Vermeidung allergischer Reaktionen vorteilhaft sein sollten. Weiterhin sind die erfindungsgemäßen Drug-Delivery-Systeme einfacher herzustellen, da weder eine zusätzliche Funktionalisierung einzelner Komponenten noch eine spezifische Verknüpfung zwischen Träger, Signalstoff und/oder Wirkstoff erfolgen muss. Zudem können so einfach unterschiedliche Signalstoffe und Wirkstoffe miteinander kombiniert werden ohne das ein neuer Wirkstoff entsteht, wie dies z.B. bei der kovalenten Verknüpfung des Tat-Proteins (Signalstoff) mit einem p53-Protein (Wirkstoff) (Tat-p53 Fusionsprotein) der Fall wäre. Darüber hinaus muss auch keine Freisetzung des Wirkstoffes, z.B. über eine proteolytische oder enzymatische Abspaltung vom Träger und/oder dem Signalstoff, erfolgen. Ein weiterer Vorteil der beschriebenen Drug-Delivery-Systeme ist darin zu sehen, dass unterschiedliche Signalstoffe und Wirkstoffe miteinander in einem Transportsystem formuliert werden können. Auch die Mengen der Signalstoffe und Wirkstoffe mit denen die Transportpartikel beladen werden ist frei wählbar und kann somit der jeweiligen Indikation ohne Probleme angepasst werden.

Insbesondere durch die Verwendung dendritischer Trägerpolymere kann über die Art und Anzahl funktioneller Gruppen, insbesondere von polaren Gruppen, im Träger die Beladungskonzentration der Partikel mit einem Wirkstoff und einem Signalstoff eingestellt werden, dabei können auch ungewöhnlich hohe Beladungskonzentrationen von über 20 Gew.-% an Wirk- und Signalstoff bezogen auf das Gesamtgewicht der Partikel erreicht werden. Darüber hinaus kann über die Anzahl der funktionellen Gruppen auch die Menge an freigesetztem Wirkstoff pro Zeiteinheit kontrolliert werden.

In einer bevorzugten Ausführungsform werden die von Lactoferrin abgeleiteten Peptide als Signalstoffe in das beanspruchte Drug-Delivery-System eingearbeitet, wobei zur Formulierung negativ geladene bzw. gut polarisierbare Träger benutzt werden. Alternativ können auch Träger mit einer positiven Nettoladung unter Verwendung eines negativ geladenen Formulierungshilfsstoffes, wie z.B. mittels Nukleinsäurefragmente, eingesetzt werden.

Folglich sind Drug-Delivery-Systeme enthaltend
a) ein von Lactoferrin abgeleitetes zellpenetrierendes Signalpeptid (CPP),
b) einen pharmazeutischen Wirkstoff und
c) einen polymeren Träger aus verzweigten oder vernetzten Homo- oder Heteropolymere aus Kohlenhydraten (Polysacchariden); aus Polyestern; aus Polyethern; aus Polyolen, aus Polyolefinen, aus Polyalkylenglykolen, aus Polyamiden, aus Polyacetalen, aus Polyacrylaten; aus Polyacetaten, aus Polyurethanen, aus siliziumorganischen Polymeren, aus Epoxidharzen, aus Polythiolen, aus Polycarbonaten, aus Polycaprolactonen, aus Polyglycoliden, aus Polylactiden, aus Polylactid-co-glycoliden oder aus Polytartraten,
von besonderen Interesse.

Solche Formulierungen sind insbesondere für den transzellularen Transport von pharmazeutischen Wirkstoffen durch das Darmepithel geeignet. Dementsprechend ist die Kombination von Lactoferrin abgeleiteten CPPs und Lebertherapeutika, wie z.B. bestimmter Cytostatika, Nucleosidanaloga oder α-Liponsäure, Interferon, Lamivudin, Kortikoiden, Azathioprin, Chlorambucil, Colchizin, Methotrexat, Ursodeoxycholsäure, Naloxon, Amphotericin B, Fluconazol, Albendazol, besonders bevorzugt.

Die Applikation der partikulären Drug-Delivery-Systeme kann z.B. oral, pulmonal, sublingual, buccal, nasal, okular oder gastrointestinal erfolgen. Hierbei sind insbesondere die orale und intravenöse Verabreichungsform von besonderem Interesse. Dazu können die Transportpartikel ohne Funktionsverlust zusätzlich mit handelsüblichen Gallenik-Substanzen, wie z. B. Eudragit®, verkapselt werden.

Die Herstellung der partikulären Drug-Delivery-Systeme kann mittels verschiedener Methoden durchgeführt werden. Hierbei hat sich das Koazervationsverfahren neben der Sprühtrocknung, den Hochdruckverfahren mit komprimierten Gasen und der Lösungsmittelverdampfung als eine bevorzugte Methode herausgestellt. Prinzipiell kann der Wirkstoff und der Signalstoff gemeinsam oder in getrennten Arbeitschritten zu den erfindungsgemäßen Drug Delivery Systeme formuliert werden.

Bei der Koazervation handelt es sich um eine erzwungene Phasentrennung zur Ausfällung von Kolloiden bzw. zur Herstellung von Partikeln. Dies kann durch verschiedene äußere Stimuli, wie Temperatur, pH, Salzlösungen oder nicht-Lösemittel, ausgelöst werden. Zum Schluss werden die entstandenen Partikel durch Wärme, Vernetzung, Lösemittelentzug oder Trocknung verfestigt. Das Koazervationsverfahren ist sehr vielfältig und kann mit verschiedenen Polymeren durchgeführt werden. Dabei können die Wandstärke und die Größe der Partikel sowie der Beladungsgrad eines (aktiven) Wirkstoffes frei variiert werden. Dabei kann auch das Freisetzungsprofil des verkapselten Wirkstoffes beliebig eingestellt werden. Das Koazervationsverfahren ist ein sehr effizientes Verfahren zur Herstellung von partikulären Formulierungen, da es eine hohe Ausbeute, eine hohe Beladungsrate und eine gute Reproduzierbarkeit erlaubt.

Man unterscheidet zwischen einfacher und komplexer Koazervation, sowie zwischen wässriger und organischer Phasentrennung (Arshady, R..Microspheres and microcapsules, a survey of manufacturing techniques, Part II, Coacervation, Polymer Engineering and Science, 30(15), 905, 1990). Bei der einfachen Koazervation kommt eine kolloidale Komponente, z. B. Gelatine, und bei der komplexen Koazervation zwei gegensätzlich geladene kolloidale Komponenten, z.B. Gelatine und Gum Arabicum, zum Einsatz. Das Prinzip der Koazervation besteht darin, dass z. B. eine wässrige Gelatinelösung durch Zugabe von Ethanol in ein Zweiphasen-System überführt wird, das aus einer Gelatine-reichen (Koazervat) und einer Gelatine-armen Phase besteht. Dies ist einer Polymerfraktionierung sehr ähnlich, allerdings entstehen in diesem Fall unter Einwirkung von Scherkräften Mikropartikel mit einer durchschnittlichen Größe von 2 - 5000 µm.

Die Herstellung von Mikrokapseln durch Koazervation lässt sich in drei Schritte unterteilen: (1) Erzeugung dreier nicht mischbarer Phasen, (2) Abscheidung des

Kolloids als Kapselhülle und (3) Verfestigung der Kapselhülle. Die drei nicht mischbaren Phasen bestehen aus einem äußeren Medium, einem Kernmaterial und dem Kapselhüllmaterial. Das Kapselhüllmaterial ist im äußeren Medium gelöst und das Kernmaterial ist darin dispergiert. Durch Einwirkung eines äußeren Stimulus (Temperatur, pH, Elektrolyte) wird das Kapselhüllmaterial im äußeren Medium unlöslich und legt sich an der Grenzfläche zum dispergierten Kernmaterial ab. Nach Filtration wird die Kapselhülle schließlich durch Einwirkung von Wärme, Vernetzung oder Lösemittelentzug ausgehärtet oder durch Sprühtrocknung oder Gefriertrocknung getrocknet.

Die Trocknung der partikulären Wirkstoffformulierung erfolgte besonders vorteilhaft durch Waschen der Partikel in einem besonders flüchtigen Lösungsmittel (z.B. Ethanol, Propanol, Aceton, Dichlormethan) mit anschließender Trocknung in einem Vakuum-, Teller- oder Taumeltrockner. Alternativ könnte sie auch mittels Sprüh- oder Gefriertrocknung erfolgen. Die bei der Partikelabtrennung zurückbleibende lösungsmittelreiche Phase kann in größerem Maßstab recycelt werden.

Mikropartikel können mit einem (aktiven) Wirkstoff beladen werden, d.h. dass das Kernmaterial diesem Wirkstoff entspricht. Hierbei kann es sich um hydrophobe oder hydrophile Substanzen handeln, was dazu führt, dass eine wässrige oder eine organische Phasentrennung verwendet werden muss. Im Falle der wässrigen Phasentrennung können hydrophobe Substanzen eingeschlossen/verkapselt werden. Umgekehrt wird bei hydrophilen Substanzen ' eine organische Phasentrennung benötigt, d.h. das Kolloid ist in der organischen Phase gelöst und reichert sich nach Einwirkung eines äußeren Stimulus auf der Grenzfläche zur hydrophilen Substanz an. Die Begriffe wässrige und organische Koazervation stehen somit jeweils für wasser- und öllösliche Kolloide. Die Beladung der Mikrokapseln mit dem Signal- und Wirkstoff liegt bei 0,5-70 Gew.-% und die Freisetzung der eingeschlossenen Substanz kann durch verschiedene Mechanismen eingeleitet werden: Diffusion, Auflösen des Kapselhüllenmaterials, enzymatische Degradation, etc. Folgende Kapselhüllmaterialien werden bevorzugt bei der einfachen Koazervation verwendet: Carboxymethylcellulose, Nitrocellulose, Polyvinylalkohol, Polyurethane, Schellak, Carrageenan, Alginate, Gelatine, Albumin, Kollagen, Celluloseacetat, Phthalate, Ethylcellulose, Polyglycerole, Polyester, Eudragits®, etc. Bei der komplexen Koazervation werden bevorzugt folgende Kombinationen mit Gelatine verwendet: Gelatine mit Gum Arabicum, Carbopol oder Pektin.

Eine weitere Möglichkeit, die erfindungsgemäßen partikulären Wirkstoffformulierungen herzustellen, stellen Hochdruckprozesse mit komprimierten oder überkritischen Fluiden dar (Gamse et al. in Chemie Ingenieur Technik 77 (2005) 669-680; McHugh und Krukonis in "Supercritical Fluid Extraction: Principles and Practices", Stoneham MA 1986, Fages et al., Powder Technology 141 (2004) 219-226 und Bungert et al., Ind. Eng. Chem. Res., 37, (1997)3208-3220).

Die bekanntesten Verfahren zur Partikelherstellung mit komprimierten Gasen sind der GAS (Gas AntiSolvent) Prozess, der PCA (Precipitation with a Compressed fluid Antisolvent) Prozess, der PGSS (Particles from Gas Saturated Solutions) Prozss und der RESS (Rapid Expansion of Supercritical Solutions) Prozess. Im folgenden werden diese Verfahren kurz erläutert.

Beim GAS Prozess wird eine Lösung, die Polymer, Wirkstoff, Signalstoff und Lösungsmittel enthält, in einem Autoklaven bei konstanter Temperatur vorgelegt und dann mit einem Gas als Nichtlösungsmittel beaufschlagt, so dass das Polymer und der Wirkstoff als feine Partikel ausfallen. In diesem Fall ist eine Durchmischung der Lösung/Suspension mittels eines Rührers sinnvoll, um eine Agglomeration der Partikel zu verhindern.

Die Signal- und Wirkstoffmoleküle können während des Ausfallens in der Polymermatrix eingebunden werden oder als Kern (Reservoir) vorliegen, um das sich eine polymere Beschichtung gebildet hat. Es bildet sich dann eine Suspension, die durch Filtration aufgetrennt werden kann. Durch Waschen der Partikel in einem überkritischen Fluid können Lösungsmittelreste extrahiert werden. Neben der Möglichkeit, den Prozess bei niedrigen und damit wirkstoffschonenden Temperaturen durchzuführen, kommt vor allem der Einflussnahme auf die Kinetik der Phasenumwandlung, also der Partikelbildung, eine wichtige Rolle zu. Indem sich die Übersättigung durch den zeitlichen Verlauf und die Intensität der Gaszugabe steuern lässt, kann auch die Partikelgrößenverteilung gewählt werden. In einem ersten Schritt wird die Phasentrennung eingeleitet und es bilden sich Kristallisationskeime in Form von Tröpfchen der entstehenden polymerreichen Phase, der späteren Mikropartikel. Es kommt nun darauf an, diese Tröpfchen nicht koaleszieren und wachsen zu lassen, sondern für eine möglichst rasche Extraktion des Lösungsmittels aus diesen Tropfen zu sorgen. Dann fallen die Partikel mit kleinen Durchmessern an (Gamse et al., Chemie Ingenieur Technik 77 (2005) 669-680 und Bungert et al., Ind. Eng. Chem. Res., 37, (1997) 3208-3220). Durch das gezielte Variieren dieser beiden Schritte lassen sich Partikelverteilung und Partikelgröße einstellen.

Der PCA Prozess oder auch SEDS (Solution Enhanced Dispersion by Supercritical Fluids) Prozess optimiert die beiden limitierenden Größen des GAS Prozesses, nämlich die Druckaufbaurate als Initiator zur Partikelbildung und den Stofftransport, um das Lösungsmittel aus den Tropfen zu entfernen (Gamse et al., Chemie Ingenieur Technik 77 (2005) 669-680; Fages et al., Powder Technology 141 (2004) 219-226 und Bungert et al., Ind. Eng. Chem. Res., 37, (1997) 3208-3220). Die Signalstoff-Wirkstoff-Polymerlösung aus dem Autoklaven wird hierbei verdichtet und in einer Einspritzdüse mit dem überkritischen Gas in Kontakt gebracht und in der Niederschlagseinheit zusammen verdüst. In einem abschließenden Waschvorgang wird mit dem überkritischen Fluid das Lösungsmittel durch Extraktion aus den Partikeln entfernt. Indem Lösung und überkritisches Fluid bereits kurz vor dem Versprühvorgang in der Düse zusammengeführt werden, lässt sich durch die kurze Kontaktzeit eine hohe Druckaufbaurate erreichen. Wie bereits oben ausgeführt, resultiert daraus eine hohe Übersättigung der Polymer-Wirkstoff-Signalstoff Lösung. Auf diese Weise können homogene Verteilungen, sowie geringe Partikelgrößen erreicht werden, denn nach der initiierten Phasentrennung erfolgt durch das Verdüsen eine feine Dispergierung, bei der durch die hohe spezifische Oberfläche der Polymerlösungstropfen ein verbesserter Stofftransport des Lösungsmittels in das komprimierte oder überkritische Gas erfolgen kann. Durch die überkritische Sprühtrocknung können Verdrängungskristallisation und Kristallisation durch Lösungsmittelverdampfung kombiniert werden.

Der PGSS Prozess unterscheidet sich grundlegend von den zuvor beschriebenen Hochdruckprozessen, da er ohne ein (oftmals toxisches) Lösungsmittel für das Polymer auskommt. Wie von Weidner in WO 95/21688, Gamse et al. in Chemie ingenieur Technik 77 (2005) 669-680, Fages et al. in Powder Technology 141 (2004) 219-226 und Bungert et al. in Ind. Eng. Chem. Res., 37, (1997) 3208-3220 beschrieben, wird bei diesem Prozess der Effekt der Erniedrigung der Glastemperatur eines Polymers durch das überkritische Fluid ausgenutzt. Das Polymer wird im überkritischen Fluid geschmolzen und der Wirkstoff in der Lösung dispergiert. Dabei erniedrigt sich auch die Viskosität der Polymerschmelze. Die Polymer-Gas Schmelze mit dem dispergierten Wirkstoff und Signalstoff wird in der Niederschlagseinheit über eine Düse entspannt, wobei der Düse zusätzlich noch überkritisches Gas zugeführt werden kann. Infolge der Temperaturemiedrigung durch den Joule-Thomson Effekt kühlt sich die Lösung ab, und das Polymer fällt als feines Pulver aus. Die Partikel können vom Gasstrom über einen Zyklon oder einen nachgeschalteten Elektrofilter abgetrennt werden. Auf diese Weise können die unterschiedlichen Größenfraktionen aufgetrennt werden. Der Wirkstoff kann wegen des Aufschmelzens des Polymers in der Polymermatrix dispergiert werden. Durch die Entspannung in der Düse entstehen feine, monodisperse Partikel.

Der RESS Prozess ähnelt dem PGSS Prozess, da auch in diesem Prozess kein organisches Lösungsmittel verwendet wird. Wie von Gamse et al. in Chemie Ingenieur Technik 77 (2005) 669-680, Fages et al. in Powder Technology 141 (2004) 219-226 und Bungert et al. in Ind. Eng. Chem. Res., 37, (1997) 3208-3220 beschrieben, wird zuerst das Polymer im Hochdruckautoklaven in Lösung gebracht. Der Wirkstoff und der Signalstoff gehen entweder ebenfalls in Lösung oder werden über einen Rührer dispergiert. Im Falle beladener Mikropartikel kommt einer homogenen Verteilung des Wirkstoffs und des Signalstoffes in der Schmelze eine sehr große Bedeutung zu, denn letztendlich stellt die Größe der Wirkstoffmoleküle die maßgebliche Limitierung für die Größe der Mikropartikel dar (Gamse et al., Chemie Ingenieur Technik 77 (2005) 669-680; Fages et al., Powder Technology 141 (2004) 219-226 und Bungert et al., Ind. Eng. Chem. Res., 37, (1997) 3208-3220). Die überkritische Lösung wird in einer Niederschlagseinheit bei Umgebungsdruck verdüst. Die Übersättigung der Lösung bzw. der Tröpfchen beim Entspannen tritt im Vergleich zu den oben beschriebenen Verfahren mit sehr viel größerer Geschwindigkeit ein. Durch das Entspannen sinkt in sehr kurzer Zeit die Dichte des überkritischen Fluids und damit auch die Lösungskraft auf Gas-typische Werte ab. Keimbildung und Stofftransport folgen bei diesem Prozess unmittelbar aufeinander und sind gegenüber den anderen Verfahren um ein Vielfaches optimiert (Gamse et al., Chemie Ingenieur Technik 77 (2005) 669-680; Fages et al., Powder Technology 141 (2004) 219-226 und Bungert et al., Ind. Eng. Chem. Res., 37, (1997) 3208-3220).

Mit den beschriebenen Verfahren können leicht partikuläre Drug-Delivery-Systeme mit einem bevorzugten Partikeldurchmesser von kleiner 900 µm, vorzugsweise von kleiner 500 µm erhalten werden. Mit den beschriebenen Verfahren können auch Drug-Delivery-Systeme mit einem Partikeldurchmesser zwischen 100 nm und 100 µm erzeugt werden, wobei üblicherweise Partikel mit einem Partikeldurchmesser zwischen 500 nm und 10 µm zum Einsatz kommen.

Die Wirkstoff- und Signalstoffbeladung des Trägers durch Koazervation oder Wirkstoff- und Signalstoffdispergierung in einer Trägerpolymerschmelze oder einer Trägerpolymer-reichen Lösung erfolgt bevorzugt in einem Temperaturbereich zwischen -30 °C und +100 °C, besonders bevorzugt zwischen 0 °C und 60 °C. Der Druck bei diesen Prozessen liegt bevorzugt zwischen 0,1 mbar und 20 bar, besonders bevorzugt zwischen 1 mbar und 10 bar.

Die alternative Erzeugung der erfindungsgemäßen Wirkstoffformulierung durch Sprühtrocknung, dem GAS (Gas AntiSolvent) Prozess, dem PCA (Precipitation with a Compressed fluid Antisolvent) Prozess, dem PGSS (Particles from Gas Saturated Solutions) Prozess und dem RPESS (Rapid Expansion of Supercritical Solutions) Prozess erfolgt bevorzugt im Temperaturbereich zwischen -30 °C und + 150 °C, bevorzugt zwischen 0 °C und 100 °C und bei Systemdrücken zwischen 0,1 mbar und 250 bar, bevorzugt zwischen 1 bar und 180 bar.

Als geeignete Lösungsmittel für die beschriebenen Herstellungsverfahren sind insbesondere Wasser, Alkohole, wie z. B. Ethanol oder Isopropanol, komprimiertes CO₂, komprimiertes Propan, Tetrahydrofuran, Toluol, Aceton, Benzoylperoxid, wässrige HCL-Lösung, Hexan, Essigsäure, Ethandiol, Dichlormethan, Dichlorethan und ionische Flüssigkeiten zu nennen.

Die Verkapselung der so bereitgestellten partikulären Drug Delivery Systeme, insbesondere zur prinzipiellen Oralverfügbarkeit, ist ohne Funktionsverlust mit handelsüblichen Gallenik-Substanzen, wie z.B. mit EUDRAGIT® (Degussa AG, DE), möglich.

Werden spezielle Verfahrensvarianten der Koazervation bzw. spezielle Coatingverfahren (Sprühtrocknung, Brace Microsphere Prozess, Koaxialdüse, Wirbelschichtcoating) eingesetzt, können die Wirkstoffe und Signalstoffe in mehreren Schichten mit einem oder mehreren Trägerpolymeren umhüllt bzw. überzogen vorliegen. So können z.B. die Signalstoffe in der äußern Schicht eingebettet sein, die Wirkstoffe in der inneren Schicht, so dass zuerst der Signalstoff, dann der Wirkstoff freigesetzt wird. Zudem können auch mehrere unterschiedliche Wirkstoffe in unterschiedlichen Partikelschichten aggregiert werden.

Bei der Einarbeitung von pharmazeutischen Wirkstoffen in Arzneimittel-Formulierungen, z.B. mit den eben beschriebenen Verfahren, werden immer speziellere Anforderungen an die Stabilität der Wirkstoffformulierungen, das Eigenschaftsprofil des Trägerpolymers, den Freisetzungstrigger sowie die Freisetzungskinetik gestellt. Dies liegt daran, dass die Wirkstoffe mitunter empfindlich auf ihre Umgebung reagieren (enzymatischer Abbau, Temperatur, pH-Wertänderungen), nicht löslich bzw. nicht lipophil sind. Die mit diesen Verfahren, unter Verwendung der oben beschriebenen linearen oder verzweigten oder vemetzten polymeren Trägem, hergestellten partikulären Drug-Delivery-Systemen zeigen eine besonders hohe Stabilität, wodurch insbesondere toxische, empfindliche, reaktive oder instabile pharmazeutische Wirkstoffe zusammen mit den Signalstoffen kontrolliert freigesetzt bzw. stabilisiert werden können. Verwendet man nun verzweigte oder vernetzte Trägerpolymere, bevorzugt dendritische Polymere und besonders bevorzugt Polyestergruppen-enthaltende hyperverzweigte Polymere, hyperverzweigte Polyglycerole,Polysaccharide oder PAMAM-Dendrimere als Trägermaterialien, für biologisch aktive Wirkstoffe und Signalstoffe können die beschriebenen Nachteile gemindert oder ganz behoben werden.

Des weiteren können insbesondere die dendritischen Trägerpolymere, wohl aufgrund ihrer für Polymere vergleichsweise niedrigen Schmelz- und Lösungsviskositäten, die Verkapselungsverfahren mit deutlich reduzierten Mengen an Lösungsmitteln bzw. komprimierten Gasen betrieben werden. Das dendritische Polymer fungiert dabei selbst als Lösungsmittel/Dispergiermittel. Durch das verringerte Lösungsmittelaufkommen wird die Herstellung der partikulären Transportsysteme sicherer, insbesondere wird die Freisetzung explosionsfähiger oder gesundheitschädlicher Dämpfe deutlich verringert.

Die kontrollierte Wirkstofffreisetzung kann insbesondere durch die Dicke der Trägerpolymerlage, die den Wirkstoff bzw. den Signalstoff umgibt, die Art und Anzahl der funktionellen Gruppen im Trägerpolymer und durch die Art des Verkapselungverfahrens beeinflusst werden. Der Freisetzungzeitraum ist umso länger, je dicker die Trägerpolymerhülle ist. Die Dicke des Trägers lässt sich, neben der Variation der Prozessparameter (pH-Wert, Temperatur, Lösungsmittel) insbesondere durch die Veränderung der Polymerkonzentration in der Ausgangsmischung erreichen. Durch die Verwendung von dendritischen Polymeren als Träger kann die Beladung des Trägers mit Wirk- und Signalstoffen auf bis zu 70 Gew.-% gesteigert werden, so dass sich besonders lange Freisetzungszeiten bei hohen freigesetzten Wirkstoffmengen erzielen lassen. Neben der Dicke der Trägerpolymerhülle entscheidet der Funktionalisierungsgrad über bzw. die Hydroxyzahl über den Freisetzungszeitraum. Soll die Freisetzung der Wirkstoffe in polaren Medien erfolgen, so ist die Freisetzung umso langsamer, je weniger freie polare Gruppen, z. B. OH-Gruppen, im Trägerpolymeren enthalten sind. Die Zahl der freien OH-Gruppen kann wiederum durch Veresterung mir Fettsäuren beeinflusst werden.

Die Freisetzung der Signal- und Wirkstoffe aus dem partikulären Drug-Delivery-System erfolgt durch unterschiedliche Mechanismen, wie z.B. durch enzymatischen Abbau des Trägerpolymers, durch hydrolytische Prozesse, pH-Wert-Änderungen oder Temperaturänderungen. Für die zielgerichtete Freisetzung pharmakologischer Wirkstoffe ist vor allem der enzymatische Abbau des Trägers von Interesse. Besonders geeignete polymere Träger sind diesbezüglich Estergruppen-tragende Polymere, insbesondere verzweigte oder vernetzte, bevorzugt dendritische Polyester.

Die partikulären Drug-Delivery-Systeme können weiterhin übliche Hilfsstoffe, wie z.B. Stabilisatoren, Tenside, Öle, Wachse, Pflanzen-, Hefe-, Algenextrakte, Aminosäuren, Aminosäurenderivate, Vitamine und deren Derivate, bioaktive Lipide, wie Cholesterol, Ceramide, Pseudoceramide, Antioxidantien, Konservierungsmittel, Farbstoffe und Pigmente enthalten.

### Beschreibung der Figuren:

Fig. 1a zeigt beispielhaft ein Träger-Wirkstoff-Signalstoff-Aggregat, bestehend aus einer unspezifisch eingelagerten, unmodifizierten Barrieren Signal-Substanz (1), z.B. dem Tat-Protein, einem unspezifisch eingelagerten, unmodifizierten Wirkstoff (2), wobei die Signalsubstanz und der Wirkstoff in zwei Schichten mit einem verzweigten polymeren Träger (3) koazerviert sind. Die Wirksoffformulierung ist darüber hinaus mit einer oral-digestiven Schicht (4) umgeben, z.B. von Eudragit®.
   In Fig. 1b wird die Freisetzung des Signalstoffes (8) in der extrazellularen Matrix (5) gezeigt, wodurch die Aufnahme (9) des Transportsystems durch die Zellmembran (7) in das Zellplasma (6) erfolgt. Fig. 1c zeigt dann die folgende Freisetzung (10) des Wirkstoffes in der Zelle.
Fig. 2 zeigt schematische Beispiele für ein dendritische Trägermaterial, Fig. 2a zeigt beispielhaft ein Dendrimer, Fig. 2b ein hyperverzweigtes Polymer.

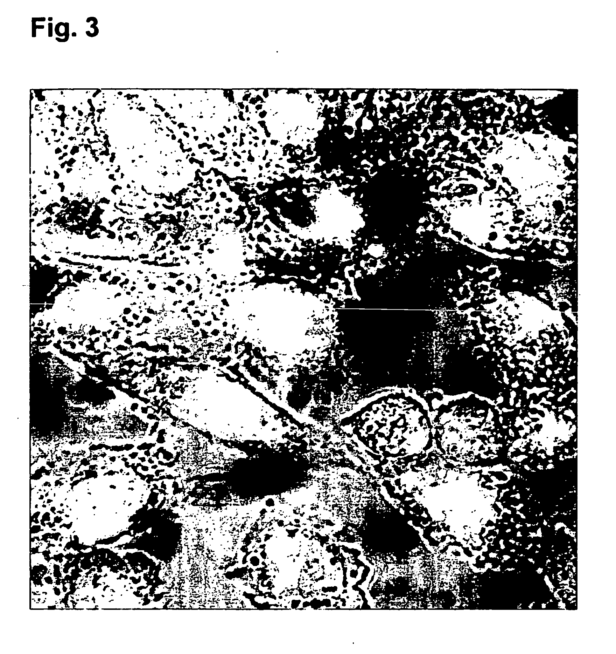

Fig. 3 zeigt eine fluoreszenzmikroskopische Aufnahme von CHO-Zellen, die mit erfindungsgemäßen Drug-Deliver-Systemen behandelt wurden, wobei als Signalstoff das Tat-Peptid und als Wirkstoffanalogon Carboxyfluoreszin (Marker) eingesetzt wurde. Man erkennt die zielgerichtete Aufnahme des Markers in die CHO-Zellen, außerhalb der CHO-Zellen ist nach 30-minütiger Inkubation praktisch keine Fluoreszenz mehr erkennbar. Dieser Effekt unterstützt die These, dass mit dem beschriebenen Drug-Delivery-System, auch mittels nicht spezifisch gebundener extrazellulärer Stimuli Wirkstoffe durch eine Zellmembran (Barriere) transportiert werden können ("Hopping").

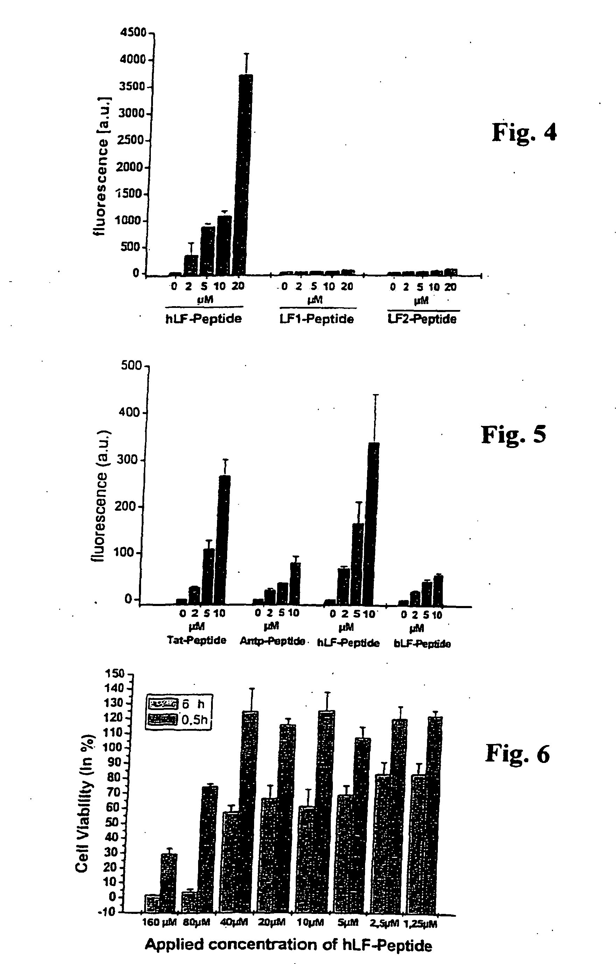

Fig. 4 zeigt die mittels Durchflusscytometrie gemessene Konzentrationsabhängigkeit der Aufnahme verschiedener peptidischer Signalstoffe in HeLa-Zellen (Tat-Peptid, Antp-Peptid, hLF-Peptid (mit einer Aminosäuresequenz gemäß den Positionen 38 bis 59 entsprechend SEQ ID No. 1), bLF-Peptid (mit einer Aminosäuresequenz gemäß den Positionen 33 bis 50 entsprechend SEQ ID No. 2)).
Fig. 5 zeigt die mittels Durchflusscytometrie bestimmte Aufnahme von Fluorescein-markierten hLF-Peptiden (mit einer Aminosäuresequenz gemäß.den Positionen 38 bis 59 entsprechend SEQ ID No. 1) im Vergleich zu verkürzten Peptiden LF1 und LF2 (mit einer Aminosäuresequenz gemäß den Positionen 40 bis 55 entsprechend SEQ ID No. 1 (LF1) bzw. mit einer Aminosäuresequenz gemäß den Positionen 40 bis 50 entsprechend SEQ ID No. 1 (LF2)).
Fig. 6 zeigt das Ergebnis von Toxizitätsuntersuchungen an HeLa -Zellen, bei 0,5-stündiger bzw. 6-stündiger Inkubation mit unterschiedlichen Konzentrationen an hLF-Peptiden (mit einer Aminosäuresequenz gemäß den Positionen 38 bis 59 entsprechend SEQ ID No. 1).

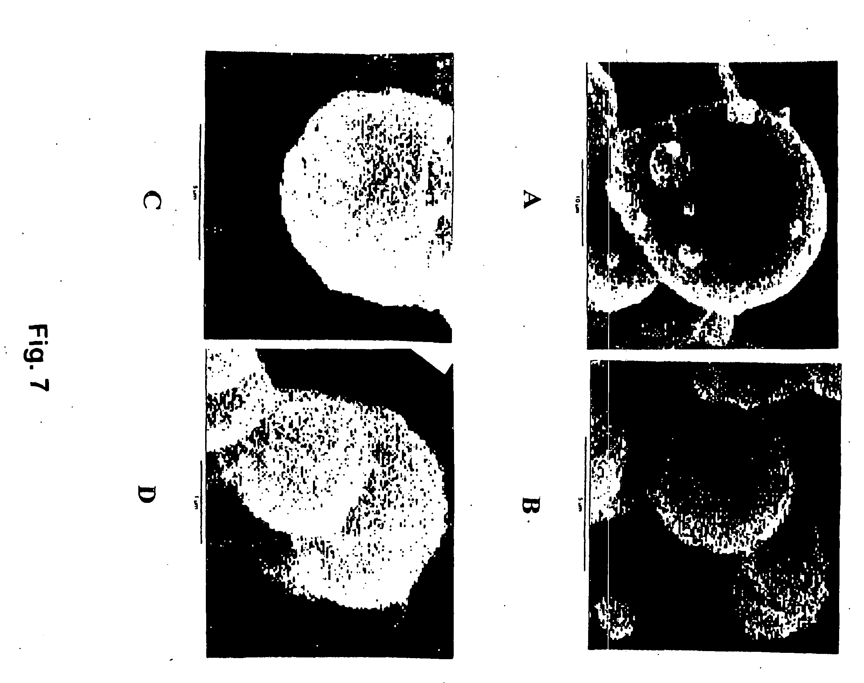

Fig. 7 zeigen partikulare Drug-Delivery-Systeme, die alpha-Liponsäure (Fig. 7 A und B) bzw. ein von humanem Lactoferrin abgeleitetes Signalpeptid und alpha-Liponsäure enthalten.

Ausführungsbeispiele:
In den Ausführungsbeispielen werden die folgenden Abkürzungen verwendet:
   - Boc: *tert*.-Butyloxycarbonyl
   - DIPEA: Diisopropylethylamin
   - DMF: Dimethylformamid
   - EDT: Ethandithiol
   - Fmoc: 9-Fluorenylmethoxycarbonyl
   - HBTU: 2-(1H-Benzotriazol-1-yl)-1,1,3,3-tetramethyluroniumhexa- fluorophosphat
   - HPLC: Hochdruckflüssigkeitschromatorgraphie
   - Pmc: 2,2,5,7,8-Pentamethylchromansulfonyl
   - tBu: *tert*.-Butyl
   - TES: Triethylsilan
   - TFA: Trifluoressigsäure
   - Trt: Triphenylmethyl

### Beispiel 1: Herstellung eines peptidischen Signal-Adjuvants.

Mit einer Fmoc/tBu Strategie wird ein naturidentisches Signalpeptid mit der Sequenz Tyr-Gly-Arg-Lys-Lys-Arg-Arg-Gln-Arg-Arg-Arg (Tat-Sequence) hergestellt, wobei die alpha-Aminofunktion der eingesetzten L-Aminosäuren Fmoc-geschützt ist und die Seitenketten der trifunktionellen Aminosäuren durch folgende Schutzgruppen geschützt sind: Boc bei Lysin und Tryptophan; tBu bei Asparaginsäure, Glutaminsäure, Serin, Threonin und Tyrosin; Pmc bei Arginin; Trt bei Cystein, Histidin, Asparagin und Glutamin.

Das Peptid wird automatisiert auf einem Syroll-Peptidsynthesizer (MultiSynTech, Witten, DE) hergestellt. Dazu werden jeweils 70 mg Fmoc-Gly-Trityl-Harz (Beladung 0,85 mmol/g; 59,5 µmol) in 5 ml Kunststoffspritzen mit Kunststofffritten vorgelegt. Die seitenkettengeschützten Fmoc-Aminosäuren werden in 0,4 M DMF gelöst, als Aktivierungsreagenzien wird 0,4 M HBTU in DMF und 0,8 M DIPEA in DMF eingesetzt, die Abspaltung der temporären Fmoc-Schutzgruppe erfolgt mit 40% Piperidin in DMF. Die Kupplung der Aminosäuren, die Abspaltung der Fmoc-Schutzgruppe und die Waschschritte erfolgt unter Rühren mit einem Magnetrührer.

Syntheseprotokoll: Das Harz wird zuerst mit 2 ml DMF 5 min gequollen und abgesaugt und danach noch dreimal mit 2 ml DMF gewaschen. Zur Abspaltung der Fmoc-Schutzgruppe des primären Fmoc-Glycins wird das Harz mit 1,2 ml 40% Piperidin/DMF 15 min versetzt, abgesaugt und nochmals mit 1,2 ml 40% Piperidin/DMF 15 min versetzt, abgesaugt und viermal mit 2 ml DMF gewaschen. Zur Kupplung werden zu dem Harz zuerst 600µl der jeweiligen Aminosäurenlösung 0,4 M in DMF (240 µmol, 4 eq), dann 600 µl 0,8 M DIPEA/DMF (480 µmol, 8 eq) und im Anschluss 600 µl 0,4 M HBTU/DMF (240 µmol, 4 eq) zugegeben. Nach 1 h wird die Kupplungslösung abgesaugt und das Harz viermal mit 2 ml DMF gewaschen. Die weiteren Kupplungszyklen werden analog der ersten Position durchgeführt, wobei, wie eben beschrieben, zuerst die Fmoc-Abspaltung erfolgt. An das gewaschene aktivierte Produkt wird dann eine weitere Fmoc geschützte Aminosäure angekuppelt und das erhaltene Kupplungsprodukt wird wiederum gewaschen. Nach Kupplung der letzten Aminosäure wird die Fmoc-Schutzgruppe wie oben angegeben abgespalten, das Harz wird dann viermal mit 2 ml DMF, 2 ml Methanol und 2 ml Dichlormethan gespült und trockengesaugt. Im Anschluss wird das erhaltene Peptid 3 h mit 1,5 ml TFA/EDT/TES/H₂O (92,5:2,5:2,5:2,5) behandelt, um die Seitenkettenschutzgruppen zu entfernen und das aufgebaute Peptid vom festen Träger abzuspalten. Nach Filtration des resultierenden Peptids wird das peptidhaltige Filtrat in einem IR-Vakuumverdampfer (TecConsult+Trading, Eggstätt, DE) auf jeweils 0,5 ml eingeengt, mit 3,5 ml eiskaltem Diethylether versetzt und 2 h bei -20°C, aufbewahrt. Das ausgefallene Peptid wird dann -abzentrifugiert und noch dreimal mit 3,5 ml eiskaltem Diethylether versetzt, aufgeschlämmt, 2 h bei -20°C aufbewahrt und wieder abzentrifugiert. Das erhaltene Peptid wird danach im Vakuum getrocknet. Das erhaltene Produkt wurde mittels HPLC und Massenspektrometrie überprüft.

Die für die Synthese benötigten Scavengerverbindungen wurden bei Fluka (Seelze, DE) erworben. Das Syntheseharz stammte von Rapp Polymere (Tübingen, DE), die seitenkettengeschützten Fmoc-Aminosäuren sowie HBTU stammten von Novabiochem (Bad Soden, DE).

### Beispiel 2: Herstellung einer partikulären Formulierung:

Dazu wird ein dendritisches Polymer (Polyamidoamin (PAMAM)-Dendrimer) mit einer Molmasse M_{w} von 6909 g/mol, einer Schmelzviskosität bei 80 °C von kleiner 3 Pa s, einem Verzweigungsgrad von 100 % und einem Durchmesser des PAMAM-Trägermoleküls von ca. 36 Å bei 100 °C in einem ersten Mischgefäß geschmolzen.

Zur Simulation eines Wirkstoffes wird Carboxyfluoreszein (Marker) verwendet. Das Carboxyfluoreszein wird zusammen mit dem in Beispiel 1 hergestellten Signalpeptid in das erste Mischgefäß hinzudosiert bis ein Markeranteil von ca. 1 Gew.-% und eine Signalpeptidanteil von ca. 1 Gew.-% bezogen auf die Polymerschmelze erreicht ist. Das Carboxyfluoreszein und das Signalpeptid werden dabei durch intensives Mischen in der Polymerschmelze dispergiert.

In einem zweiten Mischgefäß wird eine Mischung aus 2 Gew.-% Pektin (Stabilisator), 1 Gew.-% Laurylethersulfat (Emulgator) in 87 Gew.-% Wasser (Lösemittel) bei 50 °C unter Rühren vorgelegt, in die 10 Gew.-% Polymer/Marker/Signalpeptid-Dispersion aus dem ersten Mischgefäß unter fortwährendem Rühren eindosiert wird (die Gew.-% Angaben beziehen sich hier auf das Gesamtgewicht der Emulsion im zweiten Mischgefäß). Nach einer Verweilzeit von bis zu 10 Minuten sedimentieren die resultierenden partikulären Formulierungen. Die erhaltenen Partikel werden im Anschluss abfiltriert, mit dem leicht flüchtigen Lösemittel Ethanol gewaschen und dann in einem Vakuum-, Teller- oder Taumeltrockner getrocknet.

Der Grossteil der erhaltenen Partikel besitzen eine Partikelgröße zwischen 1 µm und 200 µm.

Beispiel 3: Beweis des Barrieren-Transports der partikulären Drug Delivery Systeme:

Dazu werden HeLa- bzw. CHO-Zellen in 8-Kammerdeckgläser (Nunc) in einem Dulbecco's modified eagle's Medium mit und ohne pH-Indikatoren enthaltend 10 µM der in Beispiel 2 hergestellten partikulären Drug Delivery Systeme für 30 min bei 37 °C inkubiert. Anschließend werden die Zellen mit Medium gewaschen, durch Trypsinisierung für 5 min abgelöst, in PBS suspendiert und unmittelbar im Anschluss die mittlere Fluoreszenzintensität/Zelle für insgesamt 10.000 Zellen mit einem Durchflusszytometer bestimmt (BD FACSCalibur System, Becton Dickinson, Heidelberg, DE). Lebende Zellen werden basierend auf Seitwärts- und Vorwärtsstreuung ausgewählt. Die ausgewählten Zellen zeigen eine ungestörte Morphologie.

Alle Messungen der Wirkstoff-Aufnahme werden mit lebenden Zellen mit einem inversen LSM510 Lasercanningmikroskop (Carl Zeiss, Göttingen, DE) unter Verwendung eines Plan-Apochromat 63x 1.4 N.A. Objektivs durchgeführt. Die Inkubation mit Peptid erfolgt wie für die Durchflusszytometrie beschrieben. Für die Detektion des Carboxyfluoreszeins wird die Fluoreszenz mit der 488 nm Linie eines Argon-lonen Lasers über einen HFT UV/488 Strahlteiler angeregt; die Fluoreszenz wird mit einem BP 505-550 "Bandpass"-Filter detektiert.

Fig. 3 zeigt beispielhaft die Signalentwicklung von CHO-Zellen nach 30 minütiger Inkubation unter dem Konfokal-Fluoreszensmikroskop. Es ist die gute Eindringtiefe des Fluoreszens-Signals ("Wirkstoff"-Signals) zu erkennen. Dementsprechend ist keine kovalente Verknüpfung des Signalpeptides mit dem Wirkstoff oder dem polymeren Träger nötig, um den Transport des Wirkstoffes durch die Zellmembran zu gewährleisten.

In einem weiteren Versuch wurde, analog zu den Beispielen 1 und 2, ein partikuläres Drug-Delivery System mit einer Kombination aus den fluoreszierenden Farbstoffen Cy3 (0,5 Gew.-%) und Cy5 (0,5 Gew.-%) erzeugt und damit CHO-Zellen inkubiert. Auch hier konnte eine zielgerichtete Aufnahme beider Farbstoffe in die CHO-Zellen nachgewiesen werden.

### Beispiel 4: Humanes und bovines Lactoferrin als Signalstoff

### Allgemeines:

Zellen und Reagenzien: Die verwendeten humanen HeLa -Karzinomzellen stammen aus der American Type Culture Collection (Manassas, VA, USA). HeLa -Zellen wurden in RPMI 1640 Medium mit stabilisiertem Glutamin und 2,0 g/L NaHCO₃ (PAN Biotech, Aidenbach, Deutschland) und mit 10% -igem fötalen Kälberserum (PAN Biotech) kultiviert. Chlorbromazin wurde von (Calbiochem (Bad Soden, Deutschland), 5-(N-Ethyl-N-isopropyl)amiloride (EIPA), methyl-ßcyclodextrin (MßCD) und MTT [3-(4,5-dimethylthiazol-2-yl)-2,5-diphenyltetrazoliumbromid] wurde von der Firma Sigma (Deisenhofen, Deutschland) bezogen.

Peptide: Die verwendeten Peptide wurden von EMC microcollections (Tübingen, Deutschland) synthetisiert. Die Reinheit aller Peptide wurde mittels analytischer HPLC überprüft. Die Identität der Peptide wurde durch MALDI-TOF Massenspektrometrie bestätigt. Die Reinheit aller Peptide war >95% (214 nm HPLC). Die Peptide wurde mit Carboxyfluorescein N-terminal markiert (wie in Fischer et al., Bioconjugate Chem. 14, 653-660, 2003 beschrieben).

Stocklösung der Peptide: Die Peptide wurden in DMSO aufgenommen, so dass eine 10 mM Lösung erhalten wurde. Die erhaltene Stocklösung wurde weiterhin mit PBS oder Medium versetzt. Die Peptid-Konzentration der DMSO -Stocklösung wurde anhand der Absorption von Carboxyfluorescein ermittelt. Dies erfolgte mittels UV/VIS -Spektrometrie bei einer 1:100 Verdünnung der Stocklösung mit 0.1 M Tris/HCL -Puffer (pH 8.8), die Absorption wurde bei 492 nm gemessen und es wurde ein Extinktionskoeffizienten von Carboxyflourescein von 75,000 L/(mol·cm) angenommen.

Durchflusscytometrie: Zur Bestimmung der Effizienz der Beladung mit Peptiden, wurden HeLa -Zellen mit einer Dichte von 50.000 pro "well" in einer 24-well Platte (Sarstedt, Nümbrecht, Deutschland) in RPMI 1640 -haltigem Serum eingebracht. Nach einem Tag wurden die Zellen mit Medium gewaschen und mit den Peptiden in den gewünschten Konzentrationen gelöst und in 300 µL RPMI 1640 für 30 min inkubiert. Jeder Ansatz wurde dreimal durchgeführt. Nach der Inkubation wurden die Zellen mit Medium gewaschen und mittels 5-minütiger Trypsinisierung abgelöst, in eisgekühltem PBS enthaltend 0.1 % (w/v) BSA suspendiert und sofort mittels Durchflusscytometrie bestimmt (BD FACS Calibur Systems, Becton Dickinson, Heidelberg, Deutschland). In jeder Probe wurde die Fluoreszenz von 7.000 vitalen Zellen erreicht. Vitale Zellen wurden durch Seitwärts- und Vorwärtsstreuung erfasst.

### Beispiel 4.1.: Aufnahmeeffizienz von Peptiden von humanen und bovinem Lactoferrin.

Die aus humanem oder bovinem Lactoferrin abgeleiteten Peptide wurden mittels Festphasenpeptidsynthese hergestellt. Zur Ermittlung der Aufnahme und der intrazellularen Verteilung der Peptide in lebenden Zellen wurden beide Peptide mit Carboxyflourescein am N-Terminus modifiziert. Um zu bestimmen ob die abgeleiteten Lactoferrinpeptide eine Aktivität als zellpenetrierende Peptide besitzen, wurde die zellassoziierte Fluoreszenz der mit bLF-Peptiden oder hLF-Peptiden inkubierten HeLa -Zellen mittels Durchflusscytometrie bestimmt. Zum Vergleich wurden Antp- und Tat-Peptide als bereits gut etablierte zellpenetrierende Peptide ausgewählt.

Wie in Fig. 4 gezeigt, steigt für alle vier Peptide die zellulare Fluoreszenz, gemessen mittels Durchflusscytometrie, mit steigender Peptidkonzentration an.

### Beispiel 4.2: Ermittlung von Struktur-Aktivitätszusammenhängen

Mit 22 Aminosäuren ist das hLF-Peptid ein zellpenetrierendes Peptid mittlerer Länge. Nonaarginine hat nur neun Aminosäuren, das populäre zellpenetrierende Peptid Transportan hat 27. Vier der sieben kationischen Aminosäuren und die aromatische Aminosäure liegen dabei in der Nähe des Cytosin-Restes. Im vollständigen Protein bildet der Cytosinrest eine Dislufidbrücke aus, wodurch die Domäne eine Loop-Konformation bildet. Zusätzlich wurde die zellulare Aufnahme von gekürzten Peptiden (LF1 und LF 2, Tabelle 1) getestet, denen im Vergleich zu den vollständigen Proteinen der endständige Cysteinrest fehlt.

**Tabelle 1: Primärstruktur der getesteten Peptide**

| Nr. | Peptid | Sequenz |
|---|---|---|
| 1 | Tat-Peptid | Fluo-YGRKKRRQRRR-CONH₂ |
| 2 | Antp-Peptid | Fluo-RQIKIWFQNRRMKWKK-CONH₂ |
| 3 | hLF-Peptid | Fluo-KCFQWQRNMRKVRGPPVSCIKR-CONH₂ |
| 4 | bLF-Peptid | Fluo-PEWFKCRRWQWRMKKLGA-CONH₂ |
| 5 | LF1-Peptid | Fluo-FQWQRNMRKVRGPPVS-CONH₂ |
| 6 | LF2-Peptid | Fluo-FQWQRNMRKVR-CONH₂ |

Alle Peptide wurden als Peptidamide synthetisiert. "Fluo" steht für 5(6)-Carboxyfluorescein, CONH₂ steht für den amidierten C-Terminus der Peptide. Die unmodifizierten Aminosäuresequenzen entsprechen SEQ ID No 3 - 6 (Tabelle 1 Eintrag Nr. 3 -6), die unmodifizierte Tat -Sequenz entspricht SEQ ID No. 27, die unmodifizierte Antp -Sequenz entspricht SEQ ID No. 28.

Die Ergebnisse der Durchflusscytometrie sind in Fig. 5 dargestellt. Die Aufnahme der Peptide LF1 und LF2, denen die Cysteine fehlen war nur ein zehntel der aufgenommenen Menge an hLF-Peptid, das beide Cysteinreste enthält.

### Beispiel 4.3: Cytotoxizität von hLF-Peptiden

In den oben beschriebenen Experimenten wurden hLF -Peptidkonzentrationen von um die 40 µM eingesetzt, wodurch keine cytotoxischen Effekte beobachtet wurden. Bei der Beobachtung der Peptidaufnahme in lebende Zellen wurden allerdings relativ kurze Inkubationszeiten von weniger als einer Stunde verwendet. Daher wurde zusätzlich die Auswirkung von längeren Inkubationszeiten und höheren Konzentrationen der Peptide auf die Überlebensfähigkeit der Zellen getestet. HeLa -Zellen wurden dazu mit hLF -Peptid mit einer Konzentration von 1.25 µM bis zu 160 µM für 0,5 oder 6 Std. inkubiert. Danach wurde die Vitalität der Zellen mit einem MTT -Test bestimmt. Die Ergebnisse sind in Fig. 6 zusammengefasst.

Bei Zellen, die nur 30 min mit dem Peptid inkubiert wurden, konnten keine cytotoxischen Effekte bis zu einer Konzentration von 40 µM beobachtet werden.

Nach 6 Std. war, bei einer Peptidkonzentration von 5 µM, die Überlebensfähigkeit der Zellen leicht reduziert. Bei Konzentrationen über 40 µM wurden die Zellen abgetötet.

### Beispiel 5: Beladung von Drug-Delivery-Partikeln mit und ohne humanen Lactoferrin abgeleiteten Signalpeptiden und α-Liponsäure.

Beispiel 5.1: Der eingesetzte hyperverzweigte Polyester wurde durch Hydrophobisierung eines hydrophilen hyperverzweigten Polyesters (kommerziell von der Firma Perstorp^{®} unter der Bezeichnung Boltorn H30^{®} erhältlich), der ein Gewichtsmittel des Molekulargewichts M_{w} von 3500 g/mol, eine Glastemperatur von etwa 35 °C und eine Hydroxy-Zahl von etwa 490 mg KOH/g aufwies, erhalten. Die Hydrophobisierung erfolgte durch Veresterung des hydrophilen Polymers mit einer Mischung aus Stearinsäure und Palmitinsäure .. (massenbezogenes Verhältnis Stearinsäure zu Palmitinsäure = 2 zu 1), wobei 50% der Hydroxygruppen des hydrophilen Polymers umgesetzt wurden. Das Molekulargewicht M_{w} betrug 7500 g/mol.

Zur Herstellung des Präparates wurden 20 Gew. % der α-Liponsäure (CAS: 62-46-4; kommerziell erhältlich von der Firma Degussa^{®} AG) in dem geschmolzenen Polymer bei einer Temperatur von etwa 65°C mit einem Spiral-Rührer (200 U/min) in einem ersten Mischgefäß innerhalb von 5 Minuten gelost.

In einem weiteren Mischgefäß wurde eine Mischung aus Tensiden bestehend aus 1 Gew. % Polyvinylalkohol (M = 6000 g/mol, Polisciences^{®}, Warrington, USA) und 0,1 Gew. % eines ethoxylierten Fettalkohols (Tego^{®} Alkanol L4 der Firma Degussa^{®} AG) in Wasser bei 50 °C unter Rühren vorgelegt.

Anschließend wurde die im ersten Mischgefäß hergestellte Polymerschmelze, die neben dem Polymer auch die zu verkapselnde Substanz enthielt, aus dem ersten Mischgefäß unter fortwährendem Rühren (ULTRA-TURRAX, 3.000 U/min) in das zweite Mischgefäß bei 50°C überführt. Nach einer Verweilzeit von 2 Minuten und einer Abkühlung der im zweiten Mischgefäß enthaltenen Zusammensetzung auf eine Temperatur, die 25 °C unterhalb der Schmelztemperatur des Polymers lag, bildeten sich Partikel. Mit einer Schlauchpumpe wurde die Suspension einer Zentrifuge zugeleitet, in der die Wirkstoffpartikel bei 25°C von der kontinuierlichen Phase abgetrennt wurden. Anschließend wurden die Wirkstoffpartikel in einem Vakuumtrockner bei 25°C und 10 mbar für 100 h getrocknet.

Die Partikel waren frei von unerwünschten Lösungsmitteln und bestanden aus dem hyperverzweigten fettsäuremodifizierten Polyester und ca. 4 Gew. % α-Liponsäure, bezogen auf die Partikelmasse.

Der Partikelgehalt an α-Liponsäure wurde nach Extraktion mit Methanol bzw. Methanol/Wasser mittels HPLC bestimmt, es waren 5.4 Gew.-% α-Liponsäure, (Thioctsäure) enthalten.

Eine Probe der so erhaltenen Partikel wurde mit dem Signalpeptid aus Beispiel 4, Tabelle 1, Nr. 3 ca. 30 min in einer Acetonitril/Wasser-Lösung gequollen, über Filter abgesaugt und das äußerlich trockene fluoreszierende Polymermaterial wurde anschließend auf einem Tantalträger ein Papierfilterstreifen mit dem beladenen Polymermaterial mittels Klebeband fixiert und am Hochvakuum über Nacht getrocknet, um Acetonitrilspuren zu entfernen.

Ein Teil der Proben wurde getrocknet und auf einer Graphitklebefolie fixiert und kontaktiert. An dem Probenmaterial wurde die Morphologie durch rasterelektronenmikroskopische Aufnahmen dargestellt: Die entsprechenden Aufnahmen sind in Fig. 7 dargestellt. In den Figuren 7 A und 7 B sind elektronenmikroskopische Aufnahmen der mit α-Liponsäure beladenen Partikel gezeigt. Die Figuren 7 C und 7 D zeigen elektronenmikroskopische Aufnahmen der mit α-Liponsäure und dem Signalpeptid beladenen Partikel.

### Aufnahmebedingungen:

| | |
|---|---|
| Mikroskop: | Jeol JSM 6400 |
| Beschleunigunsspannung: | 20 KV |
| Arbeitsabstand: | 15mm |

Die erhaltenen Partikel (mit und ohne Signalpeptidbeladung) wurden mittels oberflächenanalytischer Röntgenphotonenelektronenspektroskopie (XPS) (XPS-Oberflächenanalysegerät, Firma Leybold, Köln, Mg-Kante) charakterisiert. Die Ergebnisse sind in den Tabellen 2 und 3 wiedergegeben. Die Partikel ohne Peptid zeigten die atomspezifischen Signale des Trägerpolymers (C und O), die Formulierung mit Signalpeptid zeigte zusätzlich das charakteristische Stickstoff (N) Signal. Das Ergebnis zeigt, dass Träger, Signalpeptid und Wirkstoff einfach aggregiert, also nicht kovalent verknüpft, vorliegen. Das Signal peptid ist außerdem auf der Partikeloberfläche nachweisbar.

**Tabelle 2:**

| **Element** | **Atom %** | **Orbit** | **Reg** | **Range** |
|---|---|---|---|---|
| C | 90.30 | 1s | | 291.8..279.6 |
| O | 9.10 | 1s | a2 | 533.6..528.7 |
| S | 0.60 | 2p | a5 | 167.2..161 |

**Tabelle 3:**

| **Element** | **Atom%** | **Orbit** | **Reg** | **Range** |
|---|---|---|---|---|
| C | 91.17 | 1s | | 291.6..280.3 |
| N | 0.22 | 1s | a3 | 402.2..398.3 |
| O | 8.34 | 1s | a2 | 534..528.3 |
| S | 0.27 | 2p | a5 | 166..162.2 |

Beispiel 5.2: Zur Herstellung des Präparates wurden 1 Gew. % α-Liponsäure (CAS: 62-46-4; kommerziell erhältlich von der Firma Degussa^{®} AG) und 4 Gew. % Poly(DL-lactid-co-glycolid) (CAS: 26780-50-7, kommerziell erhältlich als RESOMER^{®} RG 502H von der Firma Boehringer Ingelheim) in 95 Vol.-% Acetonitril bei Raumtemperatur mit einem Blatt-Rührer (200 U/min) in einem ersten Mischgefäß innerhalb von 5 Minuten gelöst.

In einem weiteren Mischgefäß wurde 1 Gew. % eines ethoxylierten Fettalkohols (Tego^{®} Alkanol L4 der Firma Degussa^{®} AG) in Rapsöl (EAN Nr. 22112682, erhältlich von der Firma Associated Oil Packers GmbH) bei Raumtemperatur unter Rühren vorgelegt.

Anschließend wurde die im ersten Mischgefäß hergestellte Polymerlösung, die neben dem Polymer auch die zu verkapselnde Substanz enthielt, aus dem ersten Mischgefäß unter fortwährendem Rühren (Propeller-Rührer, 500 U/min) in ein zweites Mischgefäß bei Raumtemperatur überführt.

Nach einer Verweilzeit von 3 Stunden war das organische Lösungsmittel verdampft und es bildeten sich Partikel. In demselben Mischgefäß wurde das Pflanzenöl mit n-Hexan (im Massenverhältnis 1:1) gemischt und anschließend die Partikel abfiltriert. Die filtrierten Partikel wurden in einem Vakuumtrockner bei 50°C und 10 mbar für 100 h getrocknet.

Der Partikelgehalt an α-Liponsäure wurde nach Extraktion mit Methanol bzw. Methanol/Wasser mittels HPLC bestimmt, es waren 0.4 Gew.-% α-Liponsäure (Thioctsäure) enthalten.

## Patentansprüche

1. Partikuläre Drug-Delivery-Systeme basierend auf einem polymeren Träger, mindestens einem Signalstoff für den Transport durch eine biologische Barriere und mindestens einem Wirkstoff, wobei Träger, Signalstoff und Wirkstoff keine kovalenten Verknüpfungen untereinander aufweisen
**dadurch gekennzeichnet, dass**
der Signalstoff ein von Lactoferrin abgeleitetes Peptid ist, und Wirkstoffe und Signalstoffe in mehreren Schichten mit dem Trägerpolymeren umhüllt oder überzogen vorliegen, wobei die Signalstoffe in der äußeren Schicht und die Wirkstoffe in der inneren Schicht eingebettet vorliegen, wobei das von Lactoferrin abgeleitete Peptid eine Sequenz gemäß SEQ ID No. 3 oder SEQ ID No. 4 aufweist oder eine Sequenz gemäß SEQ ID No. 3 oder SEQ ID No. 4 aufweist, wobei der Methioninrest durch eine Aminosäure, ausgewählt aus der Gruppe Valin, Isoleucin, Norvalin, Leucin und Norleucin, ausgetauscht ist.

2. Partikuläre Drug-Delivery-Systeme nach Anspruch 1, **dadurch gekennzeichnet, dass** sowohl Signalstoff als auch Wirkstoff im polymeren Träger dispergiert oder koazerviert vorliegen.

3. Partikuläre Drug-Delivery-Systeme nach Anspruch 1 oder 2, **dadurch gekennzeichnet, dass** der polymere Träger mindestens ein verzweigtes oder vernetztes Polymer mit einem Anteil von über 50 Gew.-% bezogen auf das Gesamtgewicht des Trägers enthält.

4. Partikuläre Drug-Delivery-Systeme gemäß einem oder mehreren der Ansprüche 1 bis 3, **dadurch gekennzeichnet, dass** als polymerer Träger ein dendritisches Polymer eingesetzt wird.

5. Partikuläre Drug-Delivery-Systeme gemäß einem oder mehreren der Ansprüche 1 bis 4, **dadurch gekennzeichnet, dass** der polymere Träger verzweigt ist und ein Hydrogel oder ein Kammpolymer ist.

6. Partikuläre Drug-Delivery-Systeme nach einem oder mehreren der Ansprüche 1 bis 5, **dadurch gekennzeichnet, dass** als polymerer Träger ein dendritisches Polymer mit einer Molmasse von größer 1000 g/mol und/oder einer Schmelzviskosität von kleiner 3,0 Pas bei 80°C eingesetzt wird.

7. Partikuläre Drug-Delivery-Systeme gemäß einem der Ansprüche 1 bis 6,
**dadurch gekennzeichnet, dass** das Trägerpolymer ein dendritisches Trägerpoiymer ausgewählt ist aus der Gruppe der Polyamidoamin-Dendrimere, der Polypropylenimin-Dendrimere, der Polyethylenoxid-basierten Dendrimere, der Polyether-Dendrimere, der Polyamido-Dendrimere, der Polylysin-Dendrimere, der Polyarylether-Dendrimere;
und/oder ein dendritisches Polymer aus der Gruppe der Polyester, Polyesteramide, Polyether, Polyamide, Polyethylenimine; Polycaprolactone, Polyglycerine, Polyglycolide, Polylactide, Polylactid-co-glycolide, Polytartrate und Polysaccharide;
und/oder ein verzweigtes oder vernetztes Trägerpolymer ausgewählt aus der Gruppe der natürlichen und artifiziellen Kohlenhydrat Homo- oder Copolymerisaten, der natürlichen und artifiziellen Aminosäurepolymerisaten, der natürlichen und artifiziellen Nukleinsäuren, der Polyamine, der Polyimine, der Polyester, der Polyether, der Polyole, der Polyolefine, der Polyalkylenglykole, der Polyamide, der Polyacetale, der Polyacrylate, der Polyacetate, der Polyurethane, der siliziumorganischen Polymere, der Epoxidharze, der Polythiole, der Polycarbonate, der Polycaprolactone, der Polyglycolide, der Polylactide, der Polylactid-co-glycolide und der Polytartrate ist.

8. Partikuläre Drug-Delivery-Systeme gemäß einem der Ansprüche 1 bis 7,
**dadurch gekennzeichnet, dass** der Signalstoff ein unmodifizierter Signalstoff enthaltend eine Transduktionsdomäne (PTD) ist.

9. Partikuläre Drug-Delivery-Systeme nach einem oder mehreren der Ansprüche 1 bis **8, dadurch gekennzeichnet, dass** der Wirkstoff ein unmodifizierter pharmazeutischer Wirkstoff ist.

10. Verfahren zur Herstellung partikulärer Drug-Delivery-Systemen gemäß einem oder mehreren der Ansprüche 1 bis **9, dadurch gekennzeichnet, dass** die Partikel der Drug-Delivery-Systeme mittels Koazervation oder mittels einem Hochdruckverfahren unter Verwendung eines komprimierten oder überkritischen Gases in einem Temperaturbereich zwischen -30 °C und + 150 °C und einem Druck zwischen 0,1 mbar und 250 bar erzeugt werden.

11. Verwendung von verzweigten oder vernetzten polymeren Trägem zur Herstellung von partikulären Drug-Delivery-Systemen gemäß einem oder mehreren der Ansprüche 1 bis **9.**

## Claims

1. Particulate drug delivery systems based on a polymeric carrier, at least one signal substance for transport through a biological barrier and at least one active ingredient, with carrier, signal substance and active ingredient showing no covalent linkages with one another,
**characterized in that**
the signal substance is a peptide derived from lactoferrin, and active ingredients and signal substances are present enveloped or coated with the carrier polymer in a plurality of layers, wherein the signal substances are present embedded in the outer layer and the active ingredients are present embedded in the inner layer, wherein the peptide derived from lactoferrin has a sequence as shown in SEQ ID No. 3 or SEQ ID No. 4 or a sequence as shown in SEQ ID No. 3 or SEQ ID No. 4, wherein the methionine residue is replaced by an amino acid selected from the group valine, isoleucine, norvaline, leucine and norleucine.

2. Particulate drug delivery systems according to Claim 1, **characterized in that** both signal substance and active ingredient are present dispersed or coacervated in the polymeric carrier.

3. Particulate drug delivery systems according to Claim 1 or 2, **characterized in that** the polymeric carrier comprises at least one branched or crosslinked polymer with a proportion of more than 50% by weight based on the total weight of the carrier.

4. Particulate drug delivery systems according to one or more of Claims 1 to 3, **characterized in that** a dendritic polymer is employed as polymeric carrier.

5. Particulate drug delivery systems according to one or more of Claims 1 to 4, **characterized in that** the polymeric carrier is branched and is a hydrogel or a comb polymer.

6. Particulate drug delivery systems according to one or more of Claims 1 to 5, **characterized in that** a dendritic polymer having a molar mass above 1000 g/mol and/or a melt viscosity of less than 3.0 Pas at 80°C is employed as polymeric carrier.

7. Particulate drug delivery systems according to any of Claims 1 to 6, **characterized in that** the carrier polymer is a dendritic carrier polymer selected from the group of polyamidoamine dendrimers, of polypropyleneimine dendrimers, of polyethylene oxide-based dendrimers, of polyether dendrimers, of polyamido dendrimers, of polylysine dendrimers, of polyaryl ether dendrimers;
and/or a dendritic polymer from the group of polyesters, polyesteramides, polyethers, polyamides, polyethyleneimines; polycaprolactones, polyglycerols, polyglycolides, polylactides, polylactide-co-glycolides, polytartrates and polysaccharides;
and/or a branched or crosslinked carrier polymer selected from the group of natural and artificial carbohydrate homo- or copolymers, of natural and artificial amino acid polymers, of natural and artificial nucleic acids, of polyamines, of polyimines, of polyesters, of polyethers, of polyols, of polyolefins, of polyalkylene glycols, of polyamides, of polyacetals, of polyacrylates, of polyacetates, of polyurethanes, of organosilicon polymers, of epoxy resins, of polythiols, of polycarbonates, of polycaprolactones, of polyglycolides, of polylactides, of polylactide-co-glycolides and of polytartrates.

8. Particulate drug delivery systems according to any of Claims 1 to 7, **characterized in that** the signal substance is an unmodified signal substance comprising a transduction domain (PTD).

9. Particulate drug delivery systems according to one or more of Claims 1 to 8, **characterized in that** the active ingredient is an unmodified active pharmaceutical ingredient.

10. Process for producing particulate drug delivery systems according to one or more of Claims 1 to 9, **characterized in that** the particles of the drug delivery systems are generated by coacervation or by means of a high-pressure method using a compressed or supercritical gas in a temperature range between -30°C and +150°C and a pressure between 0.1 mbar and 250 bar.

11. Use of branched or crosslinked polymeric carriers for producing particulate drug delivery systems according to one or more of Claims 1 to 9.

## Revendications

1. Systèmes d'administration de médicaments sous forme de particules à base d'un support polymère, d'au moins une substance signal pour le transport à travers une barrière biologique et d'au moins une substance active, où le support, la substance signal et la substance active ne présentent pas de liaisons covalentes les uns avec les autres, **caractérisés en ce que** la substance signal est un peptide dérivé de la lactoferrine et les substances actives et les substances signal se trouvent dans plusieurs couches sous forme enrobée ou revêtue par le polymère support, les substances signal étant enrobées dans la couche extérieure et les substances actives dans la couche intérieure, le peptide dérivé de la lactoferrine présentant une séquence selon SEQ ID No. 3 ou SEQ ID No. 4 ou une séquence selon SEQ ID No. 3 ou SEQ ID No. 4, le radical méthionine étant remplacé par un acide aminé choisi dans le groupe formé par la valine, l'isoleucine, la norvaline, la leucine et la norleucine.

2. Systèmes d'administration de médicaments sous forme de particules selon la revendication 1, **caractérisés en ce que** tant la substance signal que la substance active se trouvent sous forme dispersée ou coacervée dans le support polymère.

3. Systèmes d'administration de médicaments sous forme de particules selon la revendication 1 ou 2, **caractérisés en ce que** le support polymère contient au moins un polymère ramifié ou réticulé avec une proportion de plus de 50% en poids par rapport au poids total du support.

4. Systèmes d'administration de médicaments sous forme de particules selon l'une ou plusieurs des revendications 1 à 3, **caractérisés en ce qu'**on utilise comme support polymère un polymère dendritique.

5. Systèmes d'administration de médicaments sous forme de particules selon l'une ou plusieurs des revendications 1 à 4, **caractérisés en ce que** le support polymère est ramifié et est un hydrogel ou un polymère en peigne.

6. Systèmes d'administration de médicaments sous forme de particules selon l'une ou plusieurs des revendications 1 à 5, **caractérisés en ce qu'**on utilise comme support polymère un polymère dendritique présentant une masse molaire supérieure à 1000 g/mole et/ou une viscosité de masse fondue inférieure à 3,0 Pa.s à 80°C.

7. Systèmes d'administration de médicaments sous forme de particules selon l'une ou plusieurs des revendications 1 à 6, **caractérisés en ce que** le polymère support est un polymère support dendritique choisi dans le groupe formé par les dendrimères de polyamidoamine, les dendrimères de polypropylène-imine, les dendrimères à base de poly(oxyde d'éthylène), les dendrimères de polyéther, les dendrimères polyamido, les dendrimères de polylysine, les dendrimères de polyaryléther ;
- et/ou un polymère dendritique du groupe des polyesters, polyesteramides, polyéthers, polyamides, polyéthylène-imines ; polycaprolactones, polyglycérols, polyglycolides, polylactides, polylactide-co-glycolides, polytartrates et polysaccharides ;
- et/ou un polymère support ramifié ou réticulé choisi dans le groupe formé par les homopolymères ou copolymères d'hydrates de carbone naturels et artificiels, les polymères d'acides aminés naturels et artificiels, les acides nucléiques naturels et artificiels, les polyamines, les polyimines, les polyesters, les polyéthers, les polyols, les polyoléfines, les polyalkylèneglycols, les polyamides, les polyacétals, les polyacrylates, les polyacétates, les polyuréthanes, les polymères organosiliciés, les résines époxyde, les polythiols, les polycarbonates, les polycaprolactones, les polyglycolides, les polylactides, les polylactide-co-glycolides et les polytartrates.

8. Systèmes d'administration de médicaments sous forme de particules selon l'une quelconque des revendications 1 à 7, **caractérisés en ce que** la substance signal est une substance signal non modifiée contenant un domaine de transduction (PTD).

9. Systèmes d'administration de médicaments sous forme de particules selon l'une ou plusieurs des revendications 1 à 8, **caractérisés en ce que** la substance active est une substance active pharmaceutique non modifiée.

10. Procédé pour la préparation de systèmes d'administration de médicaments sous forme de particules selon l'une ou plusieurs des revendications 1 à 9, **caractérisé en ce que** les particules des systèmes d'administration de médicaments sont obtenues par coacervation ou au moyen d'un procédé haute pression, avec utilisation d'un gaz comprimé ou surcritique dans une plage de température entre -30°C et +150°C et une pression entre 0,1 mbar et 250 bars.

11. Utilisation de supports polymères ramifiés ou réticulés pour la préparation de systèmes d'administration de médicaments sous forme de particules selon l'une ou plusieurs des revendications 1 à 9.


### SEQUENCE LISTING

<110> Eberhard Karl universität Tübingen
<120> Peptides useful as cell-penetrating peptides
<130> 205ut01.wo
<150> EP 05028755.6
   <151> 2005-12-30
<160> 30
<170> PatentIn version 3.1
<210> 1
   <211> 711
   <212> PRT
   <213> Homo sapiens
<220>
   <221> PEPTIDE
   <222> (1)..(711)
   <223> human lactoferrin
<220>
   <221> SIGNAL
   <222> (1)..(19)
   <223>
<400> 1
<210> 2
   <211> 708
   <212> PRT
   <213> Homo sapiens
<220>
   <221> PEPTIDE
   <222> (1)..(108)
   <223> bovine Lactoferrin
<220>
   <221> SIGNAL
   <222> (1)..(19)
   <223>
<400> 2
   Met Lys Leu Phe Val Pro Ala Leu Leu Ser Leu Gly Ala Leu Gly Leu
   1 5 10 15
   Cys Leu Ala Ala Pro Arg Lys Asn Val Arg Trp Cys Thr Ile Ser Gln
               20                  25                  30
   Pro Glu Trp Phe Lys Cys Arg Arg Trp Gln Trp Arg Met Lys Lys Leu
           35                  40                  45
   Gly Ala Pro Ser Ile Thr Cys Val Arg Arg Ala Phe Ala Leu Glu Cys
       50                  55                  60
   Ile Arg Ala Ile Ala Glu Lys Lys Ala Asp Ala Val Thr Leu Asp Gly
   65                  70                  75                  80
   Gly Met Val Phe Glu Ala Gly Arg Asp Pro Tyr Lys Leu Arg Pro Val
                   85                  90                  95
   Ala Ala Glu Ile Tyr Gly Thr Lys Glu Ser Pro Gln Thr His Tyr Tyr
               100                 105                 110
   Ala Val Ala Val Val Lys Lys Gly Ser Asn Phe Gln Leu Asp Gln Leu
           115                 120                 125
   Gln Gly Arg Lys Ser Cys His Thr Gly Leu Gly Arg Ser Ala Gly Trp
       130                 135                 140
   Ile Ile Pro Met Gly Ile Leu Arg Pro Tyr Leu Ser Trp Thr Glu Ser
   145                 150                 155                 160
   Leu Glu Pro Leu Gln Gly Ala Val Ala Lys Phe Phe Ser Ala Ser Cys
                   165                 170                 175
   Val Pro Cys Ile Asp Arg Gln Ala Tyr Pro Asn Leu Cys Gln Leu Cys
               180                 185                 190
   Lys Gly Glu Gly Glu Asn Gln Cys Ala Cys Ser Ser Arg Glu Pro Tyr
           195                 200                 205
   Phe Gly Tyr Ser Gly Ala Phe Lys Cys Leu Gln Asp Gly Ala Gly Asp
       210                 215                 220
   Val Ala Phe Val Lys Glu Thr Thr Val Phe Glu Asn Leu Pro Glu Lys
   225                 230                 235                 240
   Ala Asp Arg Asp Gln Tyr Glu Leu Leu Cys Leu Asn Asn Ser Arg Ala
                   245                 250                 255
   Pro Val Asp Ala Phe Lys Glu Cys His Leu Ala Gln Val Pro Ser His
               260                 265                 270
   Ala Val Val Ala Arg Ser Val Asp Gly Lys Glu Asp Leu Ile Trp Lys
           275                 280                 285
   Leu Leu Ser Lys Ala Gln Glu Lys Phe Gly Lys Asn Lys Ser Arg Ser
   290 295 300
   Phe Gln Leu Phe Gly Ser Pro Pro Gly Gln Arg Asp Leu Leu Phe Lys
   305 310 315 320
   Asp Ser Ala Leu Gly Phe Leu Arg Ile Pro Ser Lys Val Asp Ser Ala
   325 330 335
   Leu Tyr Leu Gly Ser Arg Tyr Leu Thr Thr Leu Lys Asn Leu Arg Glu
   340 345 350
   Thr Ala Glu Glu Val Lys Ala Arg Tyr Thr Arg Val Val Trp Cys Ala
   355 360 365
   Val Gly Pro Glu Glu Gln Lys Lys Cys Gln Gln Trp Ser Gln Gln Ser
   370 375 380
   Gly Gln Asn Val Thr Cys Ala Thr Ala Ser Thr Thr Asp Asp Cys Ile
   385 390 395 400
   Val Leu Val Leu Lys Gly Glu Ala Asp Ala Leu Asn Leu Asp Gly Gly
   405 410 415
   Tyr Ile Tyr Thr Ala Gly Lys Cys Gly Leu Val Pro Val Leu Ala Glu
   420 425 430
   Asn Arg Lys Ser Ser Lys His Ser Ser Leu Asp Cys Val Leu Arg Pro
   435 440 445
   Thr Glu Gly Tyr Leu Ala Val Ala Val Val Lys Lys Ala Asn Glu Gly
   450 455 460
   Leu Thr Trp Asn Ser Leu Lys Asp Lys Lys Ser Cys His Thr Ala Val
   465 470 475 480
   Asp Arg Thr Ala Gly Trp Asn Ile Pro Met Gly Leu Ile Val Asn Gln
   485 490 495
   Thr Gly Ser Cys Ala Phe Asp Glu Phe Phe Ser Gln Ser Cys Ala Pro
   500 505 510
   Gly Ala Asp Pro Lys Ser Arg Leu Cys Ala Leu Cys Ala Gly Asp Asp
   515 520 525
   Gln Gly Leu Asp Lys Cys Val Pro Asn Ser Lys Glu Lys Tyr Tyr Gly
   530 535 540
   Tyr Thr Gly Ala Phe Arg Cys Leu Ala Glu Asp Val Gly Asp Val Ala
   545 550 555 560
   Phe Val Lys Asn Asp Thr Val Trp Glu Asn Thr Asn Gly Glu Ser Thr
   565 570 575
   Ala Asp Trp Ala Lys Asn Leu Asn Arg Glu Asp Phe Arg Leu Leu Cys
   580 585 590
   Leu Asp Gly Thr Arg Lys Pro Val Thr Glu Ala Gln Ser Cys His Leu
   595 600 605
   Ala Val Ala Pro Asn His Ala Val Val Ser Arg Ser Asp Arg Ala Ala
   610 615 620
   His Val Lys Gln Val Leu Leu His Gln Gln Ala Leu Phe Gly Lys Asn
   625 630 635 640
   Gly Lys Asn Cys Pro Asp Lys Phe Cys Leu Phe Lys Ser Glu Thr Lys
   645 650 655
   Asn Leu Leu Phe Asn Asp Asn Thr Glu Cys Leu Ala Lys Leu Gly Gly
   660 665 670
   Arg Pro Thr Tyr Glu Glu Tyr Leu Gly Thr Glu Tyr Val Thr Ala Ile
   675 680 685
   Ala Asn Leu Lys Lys Cys Ser Thr Ser Pro Leu Leu Glu Ala Cys Ala
   690 695 700
   Phe Leu Thr Arg
   705
<210> 3
   <211> 22
   <212> PRT
   <213> Artificial Sequence
<220>
   <223> synthetic
   <220>
   <221> MISC_FEATURE
   <223> synthetic
<400> 3
   Lys Cys Phe Gln Trp Gln Arg Asn Met Arg Lys Val Arg Gly Pro Pro
   1 5 10 15
   Val Ser Cys Ile Lys Arg
   20
<210> 4
   <211> 18
   <212> PRT
   <213> Artificial sequence
<220>
   <223> synthetic
   <220>
   <221> MISC_FEATURE
   <223> synthetic
<400> 4
   Cys Phe Gln Trp Gln Arg Asn Met Arg Lys Val Arg Gly Pro Pro Val
   1 5 10 15
   Ser Cys
<210> 5
   <211> 16
   <212> PRT
   <213> Artificial sequence
<220>
   <223> synthetic
   <220>
   <221> MISC_FEATURE
   <223> Synthetic
<400> 5 <210> 6
   <211> 11
   <212> PRT <213> Artificial Sequence
<220>
   <223> synthetic
   <220>
   <221> MISC_FEATURE
   <223> Synthetic
<400> 6
<210> 7
   <211> 22
   <212> PRT
   <213> Artificial sequence
<220>
   <223> synthetic
   <220>
   <221> MISC_FEATURE
   <223> synthetic
<400> 7
   Lys Cys Phe Gln Trp Gln Arg Asn Val Arg Lys Val Arg Gly Pro Pro
   1               5                   10                  15
   Val Ser Cys Ile Lys Arg
               20
<210> 8
   <211> 22
   <212> PRT
   <213> Artificial Sequence
<220>
   <223> synthetic
   <220>
   <221> MISC_FEATURE
   <223> Synthetic <400> 8
<210> 9
   <211> 22
   <212> PRT
   <213> Artificial sequence
<220>
   <223> synthetic
   <220>
   <221> MISC_FEATURE
   <223> synthetic
<220>
   <221> MISC_FEATURE
   <222> (9)..(9)
   <223> norvaline
<400> 9
   Lys Cys Phe Gln Trp Gln Arg Asn Xaa Arg Lys Val Arg Gly Pro Pro
   1 5 10 15
   Val Ser Cys Ile Lys Arg
   20
<210> 10
   <211> 22
   <212> PRT
   <213> Artificial Sequence
<220>
   <223> synthetic
   <220>
   <221> MISC_FEATURE
   <223> synthetic
<400> 10
   Lys Cys Phe Gln Trp Gln Arg Asn Leu Arg Lys Val Arg Gly Pro Pro
   1 5 10 15
   Val Ser Cys Ile Lys Arg
   20
<210> 11
   <211> 18
   <212> PRT
   <213> Artificial Sequence
<220>
   <223> synthetic
   <220>
   <221> MISC_FEATURE
   <223> synthetic
<400> 11
   Cys Phe Gln Trp Gln Arg Asn Val Arg Lys Val Arg Gly Pro Pro Val
   1 5 10 15
   Ser Cys
<210> 12
   <211> 18
   <212> PRT
   <213> Artificial sequence
<220>
   <223> synthetic
   <220>
   <221> MISC_FEATURE
   <223> Synthetic
<400> 12
   Cys Phe Gln Trp Gln Arg Asn Ile Arg Lys Val Arg Gly Pro Pro Val
   1 5 10 15
   Ser Cys
<210> 13
   <211> 18
   <212> PRT
   <213> Artificial sequence
<220>
   <223> synthetic
   <220>
   <221> MISC_FEATURE
   <223> Synthetic
<220>
   <221> MISC_FEATURE
   <222> (8)..(8)
   <223> norvaline
<400> 13
   Cys Phe Gln Trp Gln Arg Asn Xaa Arg Lys Val Arg Gly Pro Pro Val
   1 5 10 15
   Ser Cys
<210> 14
   <211> 18
   <212> PRT
   <213> Artificial Sequence
<220>
   <223> synthetic
   <220>
   <221> MISC_FEATURE
   <223> Synthetic
<400> 14
   Cys Phe Gln Trp Gln Arg Asn Leu Arg Lys Val Arg Gly Pro Pro Val
   1 5 10 15
   Ser Cys
<210> 15
   <211> 18
   <212> PRT
   <213> Artificial Sequence
<220>
   <223> synthetic
   <220>
   <221> MISC_FEATURE
   <223> Synthetic
<220>
   <221> MISC_FEATURE
   <222> (8)..(8)
   <223> norleucine
<400> 15
   Cys Phe Gln Trp Gln Arg Asn Xaa Arg Lys Val Arg Gly Pro Pro Val
   1               5                   10                  15
   Ser Cys
<210> 16
   <211> 16
   <212> PRT
   <213> Artificial Sequence
<220>
   <223> synthetic
   <220>
   <221> MISC_FEATURE
   <223> synthetic
<400> 16
   Phe Gln Trp Gln Arg Asn Val Arg Lys Val Arg Gly Pro Pro Val Ser
   1               5                   10                  15
<210> 17
   <211> 16
   <212> PRT
   <213> Artificial sequence
<220>
   <223> synthetic
   <220>
<221> MISC_FEATURE
   <223> Synthetic
<400> 17
   Phe Gln Trp Gln Arg Asn Ile Arg Lys Val Arg Gly Pro Pro Val Ser
   1 5 10 15
<210> 18
   <211> 16
   <212> PRT
   <213> Artificial Sequence
<220>
   <223> synthetic
   <220>
   <221> MISC_FEATURE
   <223> Synthetic
<220>
   <221> MISC_FEATURE
   <222> (7)..(7)
   <223> norvaline
<400> 18
   Phe Gln Trp Gln Arg Asn Xaa Arg Lys Val Arg Gly Pro Pro Val Ser
   1 5 10 15
<210> 19
   <211> 16
   <212> PRT
   <213> Artificial Sequence
<220>
   <223> synthetic
   <220>
<221> MISC_FEATURE
   <223> synthetic
<400> 19
   Phe Gln Trp Gln Arg Asn Leu Arg Lys Val Arg Gly Pro Pro Val Ser
   1 5 10 15
<210> 20
   <211> 16
   <212> PRT
   <213> Artificial Sequence
<220>
   <223> synthetic
   <220>
   <221> MISC_FEATURE
   <223> synthetic
<220>
   <221> MISC_FEATURE
   <222> (7)..(7)
   <223> norleucine
<400> 20
   Phe Gln Trp Gln Arg Asn Xaa Arg Lys Val Arg Gly Pro Pro Val Ser
   1 5 10 15
<210> 21
   <211> 11
   <212> PRT
   <213> Artificial Sequence
<220>
   <223> synthetic
   <220>
<221> MISC_FEATURE
   <223> Synthetic
<400> 21
   Phe Gln Trp Gln Arg Asn Val Arg Lys Val Arg
   1 5 10
<210> 22
   <211> 11
   <212> PRT
   <213> Artificial Sequence
<220>
   <223> synthetic
   <220>
   <221> MISC_FEATURE
   <223> Synthetic
<400> 22
   Phe Gln Trp Gln Arg Asn Ile Arg Lys Val Arg
   1 5 10
<210> 23
   <211> 11
   <212> PRT
   <213> Artificial Sequence
<220>
   <223> synthetic
   <220>
   <221> MISC_FEATURE
   <223> Synthetic
<220>
   <221> MISC_FEATURE
   <222> (7)..(7)
   <223> norvaline
<400> 23
   Phe Gln Trp Gln Arg Asn Xaa Arg Lys Val Arg
   1               5                   10
<210> 24
   <211> 11
   <212> PRT
   <213> Artificial sequence
<220>
   <223> synthetic
   <220>
   <221> MISC_FEATURE
   <223> synthetic
<400> 24
   Phe Gln Trp Gln Arg Asn Leu Arg Lys Val Arg
   1               5                   10
<210> 25
   <211> 11
   <212> PRT
   <213> Artificial Sequence
<220>
   <223> synthetic
   <220>
   <221> MISC_FEATURE
   <223> synthetic
<220>
   <221> MISC_FEATURE
   <222> (7)..(7)
   <223> norleucine
<400> 25
   Phe Gln Trp Gln Arg Asn Xaa Arg Lys Val Arg
   1               5                   10
<210> 26
   <211> 66
   <212> DNA
   <213> Artificial sequence
<220>
   <223> synthetic
   <220>
   <221> misc_feature
   <223> Synthetic
<400> 26
   aaatgcttcc aatggcaaag gaatatgaga aaagtgcgtg gccctcctgt cagctgcata 60
   aagaga 66
<210> 27
   <211> 2136
   <212> DNA
   <213> Homo sapiens
<220>
   <221> misc_feature
   <223> human lactoferrin
<400> 27
   atgaaacttg tcttcctcgt cctgctgttc ctcggggccc tcggactgtg tctggctggc 60
   cgtaggagaa ggagtgttca gtggtgcgcc gtatcccaac ccgaggccac aaaatgcttc 120
   caatggcaaa ggaatatgag aaaagtgcgt ggccctcctg tcagctgcat aaagagagac 180
   tcccccatcc agtgtatcca ggccattgcg gaaaacaggg ccgatgctgt gacccttgat 240
   ggtggtttca tatacgaggc aggcctggcc ccctacaaac tgcgacctgt agcggcggaa 300
   gtctacggga ccgaaagaca gccacgaact cactattatg ccgtggctgt ggtgaagaag 360
   ggcggcagct ttcagctgaa cgaactgcaa ggtctgaagt cctgccacac aggccttcgc 420
   aggaccgctg gatggaatgt ccctacaggg acacttcgtc cattcttgaa ttggacgggt 480
   ccacctgagc ccattgaggc agctgtggcc aggttcttct cagccagctg tgttcccggt 540
   gcagataaag gacagttccc caacctgtgt cgcctgtgtg cggggacagg ggaaaacaaa 600
   tgtgccttct cctcccagga accgtacttc agctactctg gtgccttcaa gtgtctgaga 660
   gacggggctg gagacgtggc ttttatcaga gagagcacag tgtttgagga cctgtcagac 720
   gaggctgaaa gggacgagta tgagttactc tgcccagaca acactcggaa gccagtggac 780
   aagttcaaag actgccatct ggcccgggtc ccttctcatg ccgttgtggc acgaagtgtg 840
   aatggcaagg aggatgccat ctggaatctt ctccgccagg cacaggaaaa gtttggaaag 900
   gacaagtcac cgaaattcca gctctttggc tcccctagtg ggcagaaaga tctgctgttc 960
   aaggactctg ccattgggtt ttcgagggtg cccccgagga tagattctgg gctgtacctt 1020
   ggctccggct acttcactgc catccagaac ttgaggaaaa gtgaggagga agtggctgcc 1080
   cggcgtgcgc gggtcgtgtg gtgtgcggtg ggcgagcagg agctgcgcaa gtgtaaccag 1140
   tggagtggct tgagcgaagg cagcgtgacc tgctcctcgg cctccaccac agaggactgc 1200
   atcgccctgg tgctgaaagg agaagctgat gccatgagtt tggatggagg atatgtgtac 1260
   actgcatgca aatgtggttt ggtgcctgtc ctggcagaga actacaaatc ccaacaaagc 1320
   agtgaccctg atcctaactg tgtggataga cctgtggaag gatatcttgc tgtggcggtg 1380
   gttaggagat cagacactag ccttacctgg aactctgtga aaggcaagaa gtcctgccac 1440
   accgccgtgg acaggactgc aggctggaat atccccatgg gcctgctctt caaccagacg 1500
   ggctcctgca aatttgatga atatttcagt caaagctgtg cccctgggtc tgacccgaga 1560
   tctaatctct gtgctctgtg tattggcgac gagcagggtg agaataagtg cgtgcccaac   1620
   agcaacgaga gatactacgg ctacactggg gctttccggt gcctggctga gaatgctgga   1680
   gacgttgcat ttgtgaaaga tgtcactgtc ttgcagaaca ctgatggaaa taacaatgag   1740
   gcatgggcta aggatttgaa gctggcagac tttgcgctgc tgtgcctcga tggcaaacgg   1800
   aagcctgtga ctgaggctag aagctgccat cttgccatgg ccccgaatca tgccgtggtg   1860
   tctcggatgg ataaggtgga acgcctgaaa caggtgctgc tccaccaaca ggctaaattt   1920
   gggagaaatg gatctgactg cccggacaag ttttgcttat tccagtctga aaccaaaaac   1980
   cttctgttca atgacaacac tgagtgtctg gccagactcc atggcaaaac aacatatgaa   2040
   aaatatttgg gaccacagta tgtcgcaggc attactaatc tgaaaaagtg ctcaacctcc   2100
   cccctcctgg aagcctgtga attcctcagg aagtaa                             2136
<210> 28
   <211> 22
   <212> PRT
   <213> Artificial Sequence
<220>
   <223> synthetic
   <220>
   <221> MISC_FEATURE
   <223> Synthetic
<220>
   <221> MISC_FEATURE
   <222> (9)..(9)
   <223> norleucine
<400> 28
   Lys Cys Phe Gln Trp Gln Arg Asn Xaa Arg Lys Val Arg Gly Pro Pro
   1               5                   10                  15
   Val Ser Cys Ile Lys Arg
               20
<210> 29
   <211> 22
   <212> PRT
   <213> Artificial Sequence
<220>
   <223> synthetic
   <220>
   <221> MISC_FEATURE
   <223> Synthetic
<400> 29
   Lys Cys Arg Arg Trp Gln Trp Arg Met Lys Lys Leu Gly Ala Pro Ser
   1               5                   10                  15
   Ile Thr Cys Val Arg Arg
               20
<210> 30
   <211> 18
   <212> PRT
   <213> Artificial Sequence
<220>
   <223> synthetic
   <220>
<221> MISC_FEATURE
   <223> synthetic
<400> 30
   Cys Arg Arg Trp Gln Trp Arg Met Lys Lys Leu Gly Ala Pro Ser Ile
   1               5                   10                  15
   Thr Cys